# EUROPÄISCHE PATENTSCHRIFT

(11) **EP 2 585 451 B1**
(45) Veröffentlichungstag und Bekanntmachung des Hinweises auf die Patenterteilung: **01.03.2017**
(21) Anmeldenummer: 11727451.4
(22) Anmeldetag: 24.06.2011
(51) Int. Cl.: C07D 401/14, C07D 417/14, A01N 43/56, A01N 43/78, A01N 43/824

(54) **HETEROCYCLISCHE VERBINDUNGEN ALS SCHÄDLINGSBEKÄMPFUNGSMITTEL**
HETEROCYCLIC COMPOUNDS AS AGENTS FOR PEST CONTROL
COMPOSÉS HÉTÉROCYCLIQUES UTILISÉS EN TANT QU'AGENTS DE LUTTE CONTRE LES PARASITES

(30) Priorität: 24.03.2011 EP 11159576; 28.06.2010 US 359058 P; 28.06.2010 EP 10167453
(43) Veröffentlichungstag der Anmeldung: 01.05.2013
(73) Patentinhaber: Bayer Intellectual Property GmbH, 40789 Monheim (DE)
(72) Erfinder: BRETSCHNEIDER, Thomas, 53797 Lohmar (DE); FISCHER, Reiner, 40789 Monheim (DE); FÜßLEIN, Martin, 40225 Düsseldorf (DE); JESCHKE, Peter, 51467 Bergisch Gladbach (DE); KÖHLER, Adeline, 40764 Langenfeld (DE); KLUTH, Joachim, 40764 Langenfeld (DE); MÜHLTHAU, Friedrich August, 65812 Bad Soden am Taunus (DE); SATO, Yoshitaka, Tsukuba-shi Ibaraki 300-2648 (JP); VOERSTE, Arnd, 50674 Köln (DE); SHIMOJO, Eiichi, Osaka, 586-0018 (JP)
(74) Vertreter: BIP Patents
(86) Internationale Anmeldenummer: PCT/EP2011/060596
(87) Internationale Veröffentlichungsnummer: WO 2012/000896

(56) Entgegenhaltungen:
- EP-A1- 0 569 810
- EP-A2- 0 482 349
- WO-A1-01/09098
- WO-A1-03/028458
- WO-A2-2010/006713
- DE-A1- 10 014 006
- US-B1- 6 610 853

## Beschreibung

Die vorliegende Anmeldung betrifft neue heterocyclische Verbindungen, Verfahren zu Ihrer Herstellung und ihre Verwendung zur Bekämpfung von tierischen Schädlingen, zu denen Arthropoden und insbesondere Insekten zählen, sowie Zwischenprodukte zur Herstellung der neterocyclischen Verbindungen.

Bestimmte Thiazolyl-, Thiadiazolyl- und Pyrazolylverbindungen sind bereits als insektizid wirksame Verbindungen bekannt geworden (vgl. WO 2010/006713 A2).

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I), 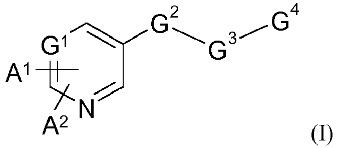 worin
- A¹ und A²: unabhängig voneinander für Wasserstoff, Halogen, Cyano, Nitro, C₁₁-C₆-Alkyl, C₃-C₆-Cycloalkyl oder C₁-C₆-Alkoxy stehen,
- G¹: für N oder C-A¹ steht,
- G²: für einen Rest aus der Reihe 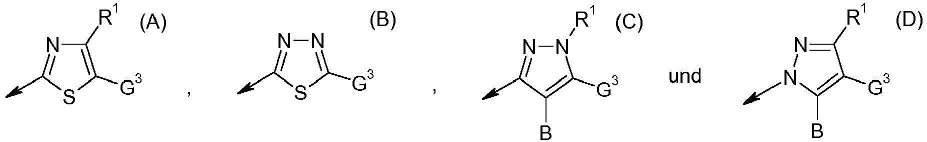
- R¹: steht, worin der Pfeil die Bindung zum benachbarten Ring markiert, im Falle der Heterocyclen (A) und (D) für Wasserstoff, Halogen, Cyano, C₁-C₆-Alkyl, C₁-C₆-Alkoxy oder C₁-C₆-Haloalkyl steht,
- R¹: im Falle von Heterocyclus (C) für Wasserstoff, C₁-C₆-Alkyl oder C₁-C₆-Haloalkyl steht,
- B: für Wasserstoff, Halogen, Cyano, Nitro, C₁-C₆-Alkyl, C₁-C₆-Haloalkyl, C₁-C₆-Alkoxy oder C₃-C₆-Cycloalkyl steht,
- G³: für jeweils gegebenenfalls durch Halogen, Cyano, Nitro, Amino, C₁-C₆-Alkylamino, Di-(C₁-C₆)-alkylamino, C₁-C₆-Alkyl, C₃-C₆-Cycloalkyl, C₁-C₆-Halogenalkyl, Hydroxy, C₁-C₆-Alkoxy, C₁-C₆-Halogenalkoxy, C₁-C₆-Alkylthio, C₁-C₆-Alkylcarbonyl, C₁-C₆-Alkoxycarbonyl, C₂-C₆-Alkenyl oder C₂-C₆-Alkinyl substituiertes 5-gliedriges Heteroaryl oder 6-gliedriges Heteroaryl steht,
- G⁴: für einen Rest aus der Reihe 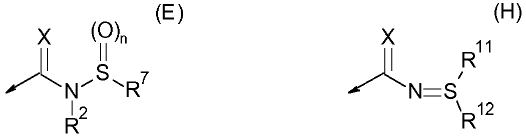 steht,
- X: für Sauerstoff oder Schwefel steht,
- n: für 1 oder 2 steht,
- R²: für einen Rest aus der Reihe Wasserstoff, C₁-C₆-Alkyl, C₁-C₆-Halogenalkyl, Cyano-C₁-C₆-alkyl, C₁-C₆-Alkoxy, C₁-C₆-Halogenalkoxy, C₂-C₆-Alkenyl, C₂-C₆-Alkinyl, C₁-C₆-Alkoxy-C₁-C₆-alkyl, jeweils gegebenenfalls durch Halogen substituiertes C₁-C₆-Alkylcarbonyl und C₁-C₆-Alkylsulfonyl, gegebenenfalls durch Halogen substituiertes C₁-C₆-Alkoxycarbonyl, gegebenenfalls durch Halogen, C₁-C₆-Alkyl, C₁-C₆-Alkoxy, C₁-C₆-Halogenalkyl und Cyano substituiertes C₃-C₆-Cycloalkylcarbonyl, oder für ein ein- oder zweiwertiges Metallion oder ein gegebenenfalls durch C₁-C₆-Alkyl oder Aryl-C₁-C₆-alkyl substituiertes Ammonium-Ion steht,
- R⁷: für einen Rest aus der Reihe jeweils gegebenenfalls durch Halogen, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl, C₃-C₆-Cycloalkyl-C₁-C₆-alkyl und C₃-C₆-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Aryl, Heteroaryl, Aryl-C₁-C₆-alkyl und Heteroaryl-C₁-C₆-alkyl oder für NR'R" steht, worin R' und R" unabhängig voneinander jeweils für einen Rest aus der Reihe Wasserstoff, C₁-C₆-Alkyl, C₁-C₆-Haloalkyl, C₃-C₆-Cycloalkyl, C₃-C₆-Cycloalkyl-C₁-C₆-alkyl, C₁-C₆-Alkoxy, C₁-C₆-Alkylcarbonyl und C₁-C₆-Alkoxylcarbonyl stehen,
- R² und R⁷: auch zusammen mit der N-S(O)ₙ Gruppe, an die sie gebunden sind, einen gesättigten oder ungesättigten und gegebenenfalls durch Halogen, C₁-C₆-Alkyl, C₁-C₆-Halogenalkyl, C₁-C₆-Alkoxy, C₁-C₆-Halogenalkoxy substituierten 5- bis 7-gliedrigen Ring bilden können, der ein oder mehrere weitere Heteroatome aus der Reihe Schwefel, Sauerstoff (wobei Sauerstoffatome nicht unmittelbar benachbart sein dürfen) und Stickstoff und/oder mindestens eine Carbonylgruppe enthalten kann,
- R¹¹ und R¹²: unabhängig voneinander für einen jeweils gegebenenfalls durch Halogen, C₁-C₆-Alkyl, C₁-C₆-Halogenalkyl, C₁-C₆-Alkoxy, C₁-C₆-Halogenalkoxy substituierten Rest aus der Reihe C₁-C₆-Alkyl, C₂-C₆-Alkenyl, C₂-C₆-Alkinyl, Phenyl und Phenyl-C₁-C₆-alkyl stehen
und wobei
Halogen ausgewählt ist aus der Reihe Fluor, Chlor, Brom und Iod,
Aryl (auch als Teil einer größeren Einheit, wie beispielsweise Arylalkyl) ausgewählt ist aus der Reihe Phenyl, Naphthyl, Anthryl, Phenanthrenyl,

Hetaryl (gleichbedeutend mit Heteroaryl, auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
sowie Salze und N-Oxide der Verbindungen der Formel (I).

Wenn G¹ für N steht, erhält man somit Verbindungen der Formel (Ia) 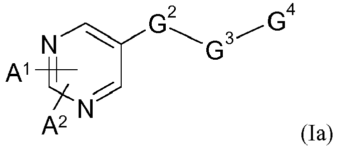 und wenn G¹ für C-A¹ steht, erhält man Verbindungen der Formel (Ib) 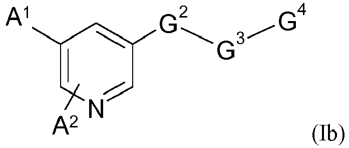 worin die übrigen Substituenten jeweils die oben genannten Bedeutungen haben.

Weiter wurde gefunden, dass man die Verbindungen der Formel (I) nach den im Folgenden beschriebenen Verfahren erhalten kann.

Verbindungen der Formel (I_{E}) können beispielsweise durch Umsetzung der Carbonsäuren der Formel (II₁) oder deren Säurechloride mit Aminderivaten der Formel (III_{E}) hergestellt werden. 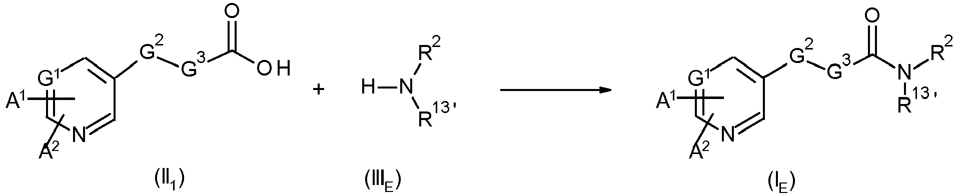 wobei R^{13'} für 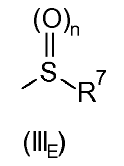 steht.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen, wie sie für andere Amide beispielsweise in DE 2221647 beschrieben sind.

Bevorzugte Substituenten bzw. Bereiche der in den Verbindungen der Formel (I) aufgeführten Reste werden im Folgenden erläutert.
- A¹: steht für Wasserstoff, Halogen oder Cyano.
- A²: steht für Wasserstoff.
- G¹: steht für N oder C-A¹.
- G²: steht für einen Rest aus der Reihe 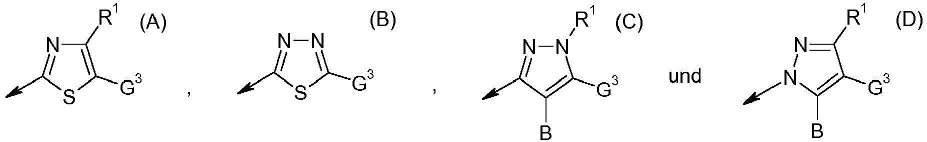 worin der Pfeil die Bindung zum benachbarten Ring markiert.
- R¹: steht für Wasserstoff oder C₁-C₄-Alkyl.
- B: steht für Wasserstoff.
- G³: steht für jeweils gegebenenfalls durch Halogen, Cyano, Nitro, Amino, C₁-C₆-Alkylamino, Di-C₁-C₆-alkylamino, C₁-C₆-Alkyl, C₃-C₆-Cycloalkyl, C₁-C₆-Halogenalkyl, Hydroxy, C₁-C₆-Alkoxy, C₁-C₆-Halogenalkoxy, C₁-C₆-Alkylthio, C₁-C₆-Alkylcarbonyl, C₁-C₆-Alkoxycarbonyl, C₂-C₆-Alkenyl oder C₂-C₆-Alkinyl substituiertes 5-gliedriges Heteroaryl oder 6-gliedriges Heteroaryl.
- G⁴: steht für den Rest 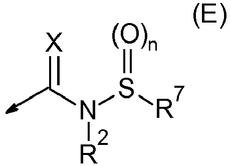 worin der Pfeil jeweils die Bindung zu G³ markiert.
- X: steht für Sauerstoff.
- n: steht für 2.
- R²: steht für einen Rest aus der Reihe Wasserstoff, C₁-C₄-Alkyl, C₂-C₄-Alkinyl, Cyano-C₁-C₄-alkyl und C₁-C₄-Alkoxy-C₁-C₄-alkyl, gegebenenfalls durch Halogen substituiertes C₁-C₄-Alkylcarbonyl oder C₁-C₄-Alkylsulfonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein ein- oder zweiwertiges Metallion oder ein gegebenenfalls durch C₁-C₄-Alkyl oder Aryl-C₁-C₄-alkyl substituiertes Ammonium-Ion.
- R⁷: steht für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, ArylC₁-C₄-alkyl und Heteroaryl-C₁-C₄-alkyl oder für NR'R", worin R' und R" unabhängig voneinander jeweils für einen Rest aus der Reihe Wasserstoff, C₁-C₄-Alkyl und C₃-C₆-Cycloalkyl-C₁-C₄-alkyl stehen.

Besonders bevorzugte Substituenten bzw. Bereiche der in den Verbindungen der Formel (I) aufgeführten Reste werden im Folgenden erläutert.
- A¹: steht für einen Rest aus der Reihe Wasserstoff, Fluor und Chlor.
- A²: steht für Wasserstoff.
- G¹: steht für N oder C-A¹, was zu Verbindungen führt, die die folgenden Strukturelemente enthalten: 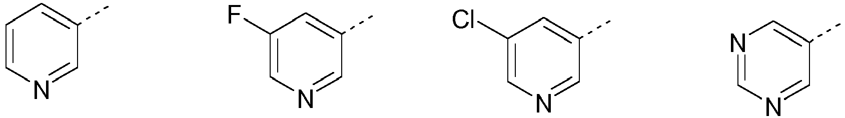 worin die gestrichelte Linie die Bindung zu G² bedeutet.
- G²: steht für einen Rest aus der Reihe 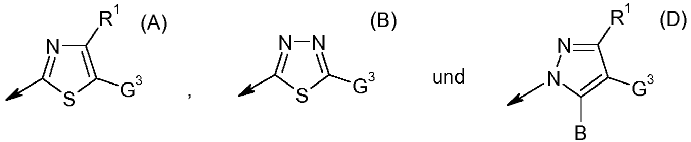 worin der Pfeil jeweils die Bindung zum benachbarten Ring markiert.
- R¹: steht für Wasserstoff oder Methyl.
- B: steht für Wasserstoff.
- G³: steht für einen gegebenenfalls durch Halogen, Cyano, Methyl, Methoxy, Trifluoromethyl, Amino oder Dimethylamino substituierten Rest aus der Reihe 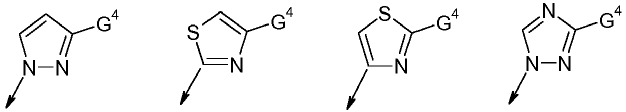 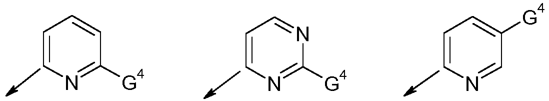 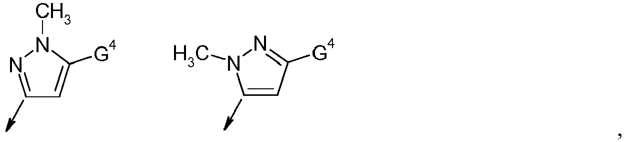 worin der Pfeil jeweils die Bindung zu G² markiert und auch G⁴ zur Veranschaulichung eingezeichnet ist.
- G⁴: steht für den Rest 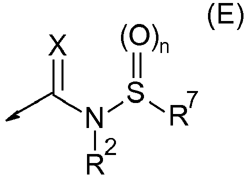 worin der Pfeil jeweils die Bindung zu G³ markiert.
- X: steht für Sauerstoff.
- n: steht für 2.
- R²: steht für einen Rest aus der Reihe Wasserstoff, C₁-C₄-Alkyl, C₂-C₄-Alkinyl, Cyano-C₁-C₄-alkyl und C₁-C₄-Alkoxy-C₁-C₄-alkyl, gegebenenfalls durch Halogen substituiertes C₁-C₄-Alkylcarbonyl oder C₁-C₄-Alkylsulfonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein ein- oder zweiwertiges Metallion oder ein gegebenenfalls durch C₁-C₄-Alkyl oder Aryl-C₁-C₄-alkyl substituiertes Ammonium-Ion.
- R⁷: steht für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können (und dabei insbesondere für 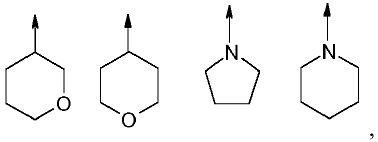 wobei der Pfeil jeweils die Bindung zum S-Atom in dem Rest (E) markiert), jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₁-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl und Heteroaryl-C₁-C₄-alkyl oder für NR'R", worin R' und R" unabhängig voneinander jeweils für einen Rest aus der Reihe Wasserstoff und C₁-C₄-Alkyl stehen und insbesondere steht R⁷ für einen Rest aus der Reihe Methyl, Ethyl, i-Propyl, CF₃, CHF₂, CH₂F, CH₂CF₃, Cyclopropyl, Dimethylamino, Diethylamino, Phenyl und Benzyl.

In den bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,

Hetaryl (auch als Teil einer größeren Einheit, wie beispielsweise Hetarylalkyl) ausgewählt aus der Reihe Pyridyl, Pyrimidyl, Oxadiazolyl, Oxazolyl, Pyrazinyl, Imidazolyl, Thiazolyl, Thiadiazolyl und Furanyl.

In den besonders bevorzugten Definitionen steht, sofern nichts anderes angegeben ist,

Aryl für Phenyl und
Hetaryl (gleichbedeutend mit Heteroaryl, auch als Teil einer größeren Einheit, wie beispielsweise Hetarylalkyl) für einen Rest ausgewählt aus der Reihe Pyrazolyl, Oxazolyl, Thiazolyl, Pyridyl, Pyrimidinyl, Pyridazinyl und Pyrazinyl.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

In den Resten (A), (B), (C) und (D), für die G² stehen kann, markiert der Pfeil jeweils die Bindung zum benachbarten Ring.

In einer hervorgehobenen Gruppe von erfindungsgemäßen Verbindungen steht G² für den Rest (A).

In einer weiteren hervorgehobenen Gruppe von erfindungsgemäßen Verbindungen steht G² für den Rest (B).

In einer weiteren hervorgehobenen Gruppe von erfindungsgemäßen Verbindungen steht G² für den Rest (C).

In einer weiteren hervorgehobenen Gruppe von erfindungsgemäßen Verbindungen steht G² für den Rest (D).

In einer weiteren hervorgehobenen Gruppe von erfindungsgemäßen Verbindungen steht X für Sauerstoff.

In einer weiteren hervorgehobenen Gruppe von erfindungsgemäßen Verbindungen steht X für Schwefel.

In einer weiteren hervorgehobenen Gruppe von erfindungsgemäßen Verbindungen steht G¹ für C-H.

In einer weiteren hervorgehobenen Gruppe von erfindungsgemäßen Verbindungen steht G¹ für C-F.

In einer weiteren hervorgehobenen Gruppe von erfindungsgemäßen Verbindungen steht G¹ für N (Stickstoff).

In einer weiteren hervorgehobenen Gruppe von erfindungsgemäßen Verbindungen steht A¹ für Wasserstoff.

In einer weiteren hervorgehobenen Gruppe von erfindungsgemäßen Verbindungen steht A² für Wasserstoff.

In einer weiteren hervorgehobenen Gruppe von erfindungsgemäßen Verbindungen steht n für 2.

In einer weiteren hervorgehobenen Gruppe von erfindungsgemäßen Verbindungen steht R¹ für Wasserstoff.

In einer weiteren hervorgehobenen Gruppe von erfindungsgemäßen Verbindungen steht R¹ für Methyl.

In einer weiteren hervorgehobenen Gruppe von erfindungsgemäßen Verbindungen steht R¹ für Fluor.

In einer weiteren hervorgehobenen Gruppe von erfindungsgemäßen Verbindungen steht G⁴ für den Rest (E).

In einer weiteren hervorgehobenen Gruppe von erfindungsgemäßen Verbindungen steht G³ für einen Pyrazolylrest.

In einer weiteren hervorgehobenen Gruppe von erfindungsgemäßen Verbindungen steht G³ für einen Thiazolylrest.

In einer weiteren hervorgehobenen Gruppe von erfindungsgemäßen Verbindungen steht G³ für einen Triazolylrest.

In einer weiteren hervorgehobenen Gruppe von erfindungsgemäßen Verbindungen steht G³ für einen Pyridinylrest.

In einer weiteren hervorgehobenen Gruppe von erfindungsgemäßen Verbindungen steht G³ für einen Pyrimidinylrest.

Eine hervorgehobene Ausführungsform der Erfindung betrifft Verbindungen der Formeln (IA) 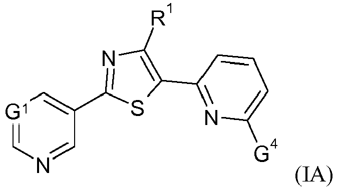 worin
- G¹: für C-H, C-F, C-Cl oder N steht,
- R¹: für Wasserstoff oder Methyl steht und
- G⁴: für den Rest (E) steht.

Eine innerhalb der Verbindungen der Formel (IA) bevorzugte Gruppe von Verbindungen sind solche, in denen G¹ für C-F steht, R¹ für Wasserstoff steht und G⁴ für den Rest (E) steht.

Eine weiter hervorgehobene Ausführungsform der Erfindung betrifft Verbindungen der Formel (IB) 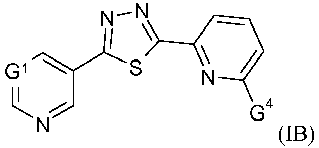 worin
- G¹: für C-H, C-F, C-Cl oder N steht und
- G⁴: für den der Rest (E) steht.

Eine weitere hervorgehobene Ausführungsform der Erfindung betrifft Verbindungen der Formel (IC) 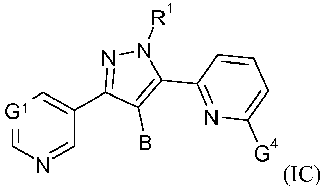 worin
- G¹: für C-H, C-F, C-Cl oder N steht,
- R¹: für Wasserstoff oder Methyl steht,
- B: für Wasserstoff steht und
- G⁴: für den Rest (E) steht.

Eine weitere hervorgehobene Ausführungsform der Erfindung betrifft Verbindungen der Formel (ID) 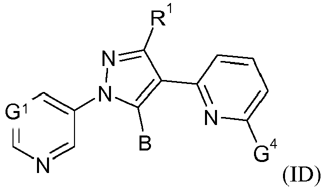 worin
- G¹: für C-H, C-F, C-Cl oder N steht,
- R¹: für Wasserstoff oder Methyl steht,
- B: für Wasserstoff steht und
- G⁴: für den Rest (E) steht.

Die Substituenten im Rest (E) in den Verbindungen der Formeln (IA), (IB), (IC) und (ID) können die vorne in der Beschreibung genannten Bedeutungen annehmen.

Die oben aufgeführten allgemeinen oder in Vorzugsbereichen aufgeführten Restedefinitionen bzw. Erläuterungen gelten für die Endprodukte der Formel (I) (und damit auch für die Verbindungen der Formeln (Ia), (Ib), (IA), (IB), (IC) und (ID)) und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt werden Verbindungen der Formel (I), in welchen eine Kombination der vorstehend als bevorzugt aufgeführten Bedeutungen vorliegt.

Erfindungsgemäß besonders bevorzugt werden Verbindungen der Formel (I), in welchen eine Kombination der vorstehend als besonders bevorzugt aufgeführten Bedeutungen vorliegt.

Die Herstellung der erfindungsgemäßen Verbindungen wird im Folgenden näher erläutert.

Die als Ausgangsstoffe benötigten Verbindungen der Formel (II₁), (II₂), (II₃), (II₄) und (II₅) in welcher G² für die Reste (A), (B) und (C) steht, können analog zu den Methoden hergestellt werden, die in WO 2010/006713 A2 beschrieben sind.

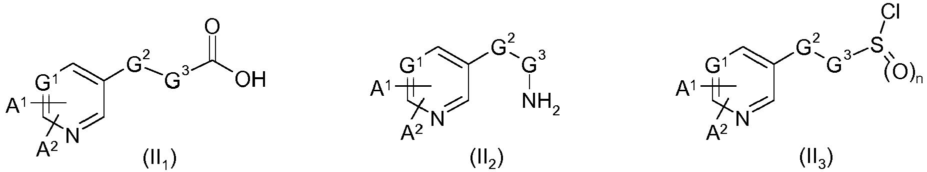

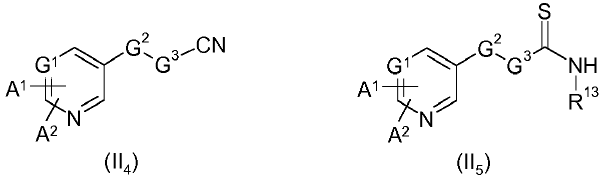

Die als Ausgangsstoffe benötigten Ausgangsverbindungen der Formel (II₁), (II₂), (II₃), (II₄) und (II₅) in welcher G² für den Rest (D) steht, können analog zu den in der Literatur beschriebenen Methoden wie folgt hergestellt werden.

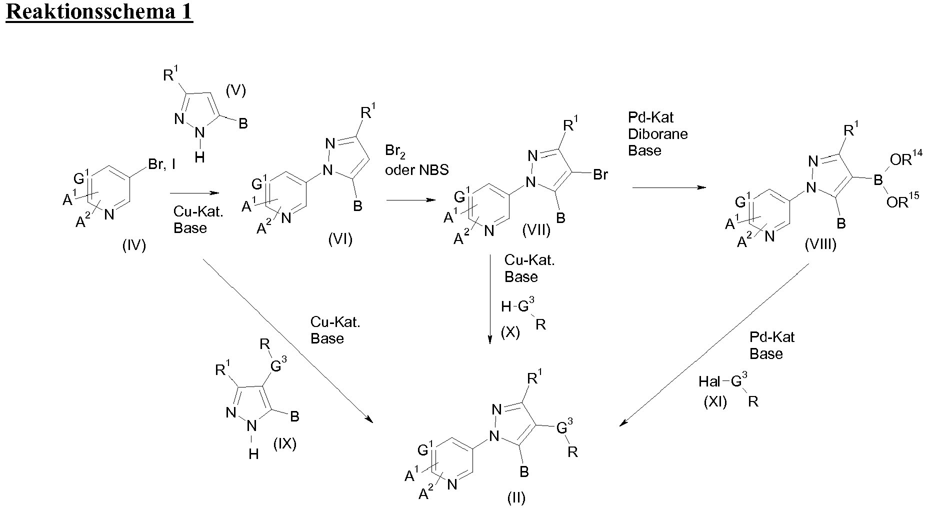

Beispielsweise erhält man durch Umsetzung eines Bromides der Formel (IV) mit einem Pyrazol der Formel (V) in Gegenwart eines Kupfer-Katalysators und einer Base wie Kaliumcarbonat die Verbindungen der Formel (VI). Siehe beispielsweise für 3-(4-Bromo-pyrazol-1-yl)-pyridin: Journal of Heterocyclic Chemistry 1981, 18, 9-14; European Journal of Organic Chemistry, 2004, 695. Aus diesen Pyrazolen der Formel (VI) werden durch Umsetzung mit Brom oder N-Bromsuccinimid die Bromide der Formel (VII) erhalten. Siehe beispielsweise für 3-(4-Bromo-pyrazol-1-yl)-pyridin: Journal of Heterocyclic Chemistry 18, 1981, 9-14. Aus den Bromiden der Formel (VII) werden durch Umsetzung mit beispielsweise Bis-Pinakolata-Diboran in Gegenwart eines Palladium-Katalysators und einer Base die Boronester der Formel (VIII) erhalten. Die benötigte Ausgangsverbindungen der Formel (II) können aus den Bromiden der Formel (VII) durch Umsetzung mit einer Verbindung der Formel (X), die einen Baustein H-G³-R darstellt, welcher ein N-H enthält, wie beispielsweise ein Pyrazol, in Gegenwart eines Kupferkatalysators und einer Base oder nach dem gleichen Verfahren durch Umsetzung der Bromide der Formel (IV) mit einem geeigneten Pyrazol der Formel (IX) erhalten werden.

Zusätzlich lassen sich erfindungsgemäße Verbindungen der Formel (I) durch Umsetzung der Boronester der Formel (VIII) mit einem Halogenid der Formel (XI) in Gegenwart eines Palladium-Katalysators und einer Base (Suzuki-Reaktion) erhalten.

Wenn R für eine geschützte Carbonsäure, beispielsweise für einen Estersteht, lässt sich die Carbonsäure (II₁) leicht durch bekannte Methoden herstellen.

Wenn R für ein geschütztes Amin steht, lässt sich das Amin (II₂) leicht durch bekannte Methoden herstellen.

Wenn R für Halogen, beispielsweise Brom steht, (analog zu WO2007/45588 A1 und US2008/318941), läßt sich das Halogen gegen ein Metall wie beispielsweise Lithium austauschen. Die Metallverbindung reagiert mit Schwefeldioxid und anschließend mit einem Chloriereungsreagenz wie Sulfurylchlorid oder N-Chlorosuccinimid zur Ausgangsverbindung (II₃).

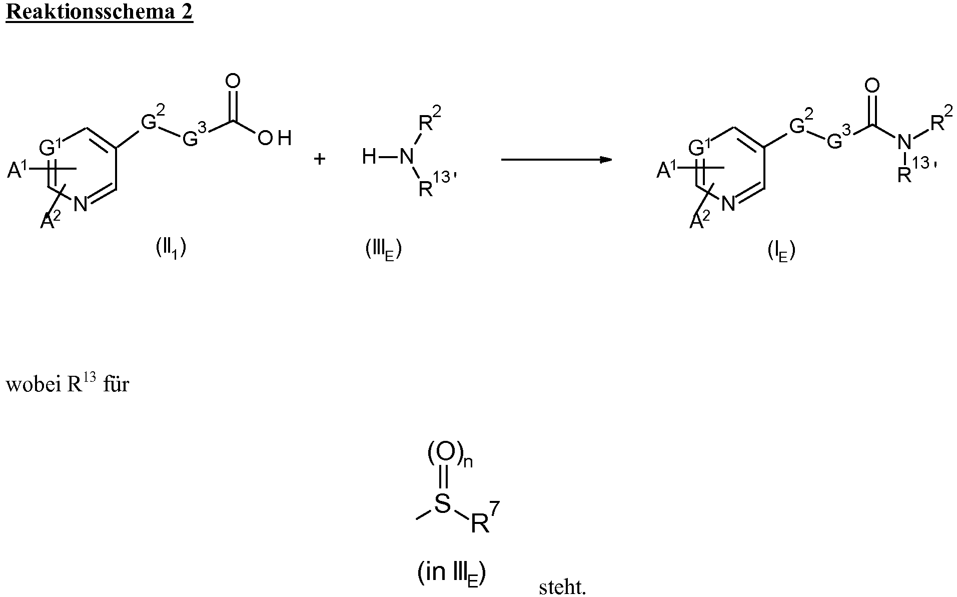

Die als Ausgangstoffe benötigten Aminderivate der Formel (III_{E}) sind bekannt oder lassen sich nach im Prinzip bekannten Methoden herstellen.

Die Säuren der Formel (II₁) lassen sich nach Aktivierung, beispielsweise zum Säurechlorid (siehe beispielsweise Bioorg & MedChem Letters 15, 4354 (2005)), oder mittels Aktivierungsreagenzien wie CDI (Carbonyldiimidazol, siehe beispielsweise Bioorg & MedChem 9, 1543 (2001), EDC (1-Ethyl-3-[3-dimethylaminopropyl]carbodiimidehydrochlorid) in Gegewart von DMAP (Dimethylaminopyridin, siehe beispielsweise J. Med. Chem. 50, 3101 (2007)), oder DCC (Dicyclohexylcarbodiimid) in Gegenwart von HOBT (1-Hydroxybenztrazole, siehe beispielsweise J. Med. Chem. 50, 3101 (2007)), mit Sulfonamiden der Formel (III_{E}) , gegebenenfalls in Gegenwart einer Base wie einem Metallhydrid (insbesondere Natriumhydrid) oder DBU (Diazabicycloundecen) zu den erfindungsgemäßen Verbindungen der Formel (I_{E}) umsetzen, in denen X für Sauerstoff steht.

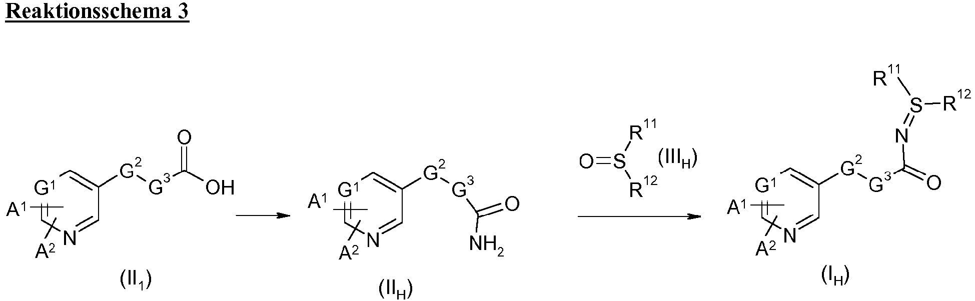

Verbindungen der Formel (I_{H}) können beispielsweise hergestellt werden durch Umsetzung der Carboxamide der Formel (II_{H}) mit Sulfoxiden der Formel (III_{H}) mittels literaturbekannter Methoden oder analogen Methoden, siehe beispielsweise WO 2008/154528 A2.

Die als Ausgangsstoffe benötigten Carboxamide der Formel (II_{H}) lassen sich aus den Säuren (II₁) oder ihren Säurechloriden mittels literaturbekannter Methoden oder analogen Methoden herstellen, beispielsweise wie in WO 2007/103755 A2 oder US 2009/203657 A1 beschrieben.

Die Sulfoxide der Formel (III_{H}) sind literaturbekannte Verbindungen.

Verbindungen der Formel (I_{E}) in denen X für Schwefel steht, lassen sich aus den entsprechenden Verbindungen der Formel (I_{E}) in denen X für Sauerstoff steht, durch Umsetzung mit einem Schwefelungsreagenz herstellen. Als Sulfidierungsmittel (Schwefelungsreagenz) werden bevorzugt Phosphorreagenzien wie beispielsweise Diphosphorpentasulfid (P₂S₅), Diphosphorpentasulfid/Pyridin (P₂S₅/P_{y}), Diphosphorpentasulfid/ Triethylamin (P₂S₅/NEt₃), Diphosphorpentasulfid/Natriumhydrogencarbonat (P₂S₅/NaHCO₃ "Scheeren's Reagenz) oder besonders bevorzugt das 2,4-Bis(4-methoxyphenyl)-2,4-dithioxo-1,3,2,4-dithiadiphosphetan "Lawesson's Reagens (LR)", 2,4-Bis(4-phenoxyphenyl)-2,4-dithioxo-1,3,2,4-dithiadiphosphetan "Belleau's Reagens (BR)" bzw. 2,4-Bis(4-phenylthiophenyl)-2,4-dithioxo-1,3,2,4-dithiadiphosphetan, eingesetzt.

N-Oxide lassen sich beispielsweise durch Umsetzung von Verbindungen der Formel (I) mit mCPBA (meta-Chlorperbenzoesäure) gewinnen. Salze von Verbindungen der Formel (I) sind zugänglich, indem man Verbindungen der Formel (I) mit Verbindungen der Formel RX umsetzt, in der X beispielsweise für Halogen wie Chlor oder Brom und R beispielsweise für einen jeweils gegebenenfalls substituierten Alkyl-, Alkenyl- oder Alkinylrest steht.

Folgende Zwischenprodukte der Formel (XII), (XV) und (XVI) sind neu und auch Gegenstand der Erfindung.

### Verbindungen der Formel (XII)

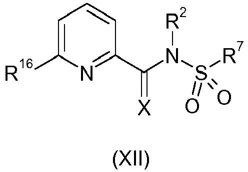

worin
R¹⁶ für Fluor, Chlor, Brom oder Iod (insbesondere für Chlor, Brom oder Iod) steht und
X, R² und R⁷ die oben angegebenen Bedeutungen haben, mit der Ausnahme, dass R⁷ nicht für Aryl stehen kann.

Insbesondere seien folgende Verbindungen der Formel (XII) genannt. 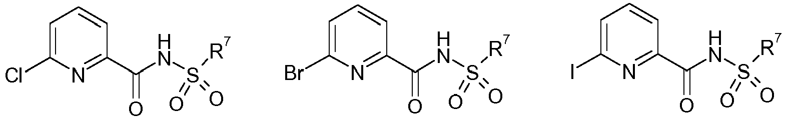 wobei R⁷ für Methyl, Ethyl, Cyclopropyl, CF₃, CH₂CF₃, Dimethylamino, Diethylamino, Phenyl oder Benzyl steht.

### Verbindungen der Formel (XV)

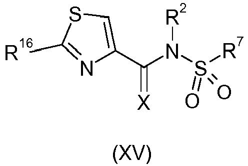

worin
R¹⁶ für Fluor, Chlor, Brom oder Iod (insbesondere für Chlor, Brom oder Iod) steht und
X, R² und R⁷ die oben angegebenen Bedeutungen haben.

Insbesondere seien folgende Verbindungen der Formel (XV) genannt. 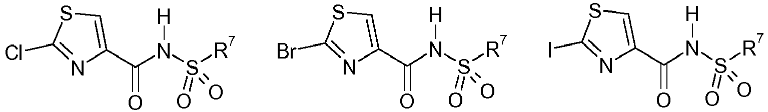 wobei R⁷ für Methyl, Ethyl, Cyclopropyl, CF₃, CH₂CF₃, Dimethylamino, Diethylamino, Phenyl oder Benzyl steht.

### Verbindungen der Formel (XVI)

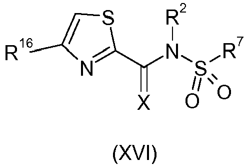

worin
R¹⁶ für Fluor, Chlor, Brom oder Iod (insbesondere für Chlor, Brom oder Iod) steht und
X, R² und R die oben angegebenen Bedeutungen haben.

### Insbesondere seien folgende Verbindungen der Formel (XVI) genannt.

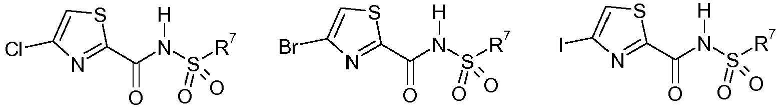

wobei R⁷ die oben angegebenen Bedeutungen hat und insbesondere für Methyl, Ethyl, Cyclopropyl, CF₃, CH₂CF₃, Dimethylamino, Diethylamino, Phenyl oder Benzyl steht.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorratsund Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl als Saatgutanwendungen als auch in Vor-, Tank- oder Fertigmischungen verwendet werden.

### Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen.
(28) Ryanodinrezeptor-Effektoren, wie beispielsweise
   Diamide, z.B. Chlorantraniliprole und Flubendiamide.
   Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und Iodmethan; desweiteren Präparate auf Basis von Bacillus firmus (I-1582, BioNeem, Votivo) sowie folgende bekannte wirksame Verbindungen: 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,-12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt aus-WO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}-benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl]-(methyl)amino}-1,3-oxazol-2(5H)-on (alle bekannt aus WO2010/005692), NNI-0711 (bekannt aus WO2002096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002096882), Methyl-2-[2-({[3-brom-1-(3-chlorpyridin-2-yl)-1H-pyrazol-5-yl]carbonyl}amino)-5-chlor-3-methylbenzoyl]-2-methyl-hydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethyl-hydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus W0O2010/069502) und N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp*., *Juglandaceae sp*., *Betulaceae sp*., *Anacardiaceae sp*., *Fagaceae sp*., *Moraceae sp*., *Oleaceae sp*., *Actinidaceae sp*., *Lauraceae sp*., *Musaceae sp.* (beispielsweise Bananenbäume und - plantagen), *Rubiaceae sp.* (beispielsweise Kaffee), *Theaceae sp*., *Sterculiceae sp*., *Rutaceae sp.* (beispielsweise Zitronen, Organen und Grapefruit); *Solanaceae sp.* (beispielsweise Tomaten), *Liliaceae sp*., *Asteraceae sp.* (beispielsweise Salat), *Umbelliferae sp*., *Cruciferae sp*., *Chenopodiaceae sp*., *Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Poaceae sp.* (z.B. Zuckerrohr), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, dass es ein interessierendes Protein oder Polypeptid exprimiert oder dass es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffe auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, dass die behandelten Pflanzen, wenn sie im Anschluss daran mit unerwünschten phytopathogenen Pilzen inokuliert wurde, einen beträchtlichen Resistenzgrad gegen diese unerwünschten phytopathogenen Pilze aufweisen. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Stressfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Stressfaktoren resistent sind. Zu den abiotischen Stressbedingungen können zum Beispiel Dürre, Kälte- und Hitzebedingungen, osmotischer Stress, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Stress- und Nicht-Stress-Bedingungen) beeinflusst werden, darunter frühe Blüte, Kontrolle der Blüte für die Produktion von Hybridsaatgut, Keimpflanzenwüchsigkeit, Pflanzengröße, Internodienzahl und - abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit. Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Stressfaktoren führt. Solche Pflanzen werden typischerweise dadurch erzeugt, dass man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, dass die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, dass die Pollenfertilität in Hybridpflanzen, die die für die Pollensterilität verantwortlichen genetischen Determinanten enthalten, völlig restoriert wird. Dies kann erreicht werden, indem sichergestellt wird, dass die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacterium sp*., die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, dass man ein Gen exprimiert, das für ein Glyphosate-Oxidoreduktase-Enzym kodiert. Glyphosatetolerante Pflanzen können auch dadurch erhalten werden, dass man ein Gen exprimiert, das für ein Glyphosate-acetyltransferase-Enzym kodiert. Glyphosatetolerante Pflanzen können auch dadurch erhalten werden, dass man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, dass man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, dass man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, dass man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, dass verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfasst im vorliegenden Zusammenhang jegliche Pflanze, die mindestens ein Transgen enthält, das eine Kodiersequenz umfasst, die für folgendes kodiert:
1) ein insektizides Kristallprotein aus *Bacillus thuringiensis* oder einen insektiziden Teil davon, wie die insektiziden Kristallproteine, die online bei:
   http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfasst, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter
   http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus*, das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfasst, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfasst, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, dass man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Stressfaktoren tolerant. Solche Pflanzen können durch genetische Transformation oder durch Selektion von Pflanzen, die eine Mutation enthalten, die solch eine Stressresistenz verleiht, erhalten werden. Zu besonders nützlichen Pflanzen mit Stresstoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein stresstoleranzförderndes Transgen enthalten, das die Expression und/oder Aktivität der für PARG kodierenden Gene der Pflanzen oder Pflanzenzellen zu reduzieren vermag;
c. Pflanzen, die ein stresstoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so dass sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellunssbeispiele

### Beispiel B: 6-[2-(5-Fluorpyridin-3-yl)-1,3-thiazol-5-yl]-N-[(trifluormethyl)sulfonyl]pyridin-2-carboxamid

### Stufe 1: 3-Fluor-5-(1,3-thiazol-2-yl)pyridin

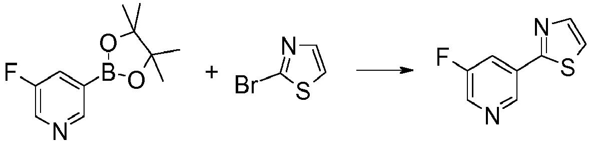

2,0g (8,97 mmol) des Pyridyl-boresters, 1,47 g (8,97 mmol) des 2-Bromthiazols, 197 mg (0,27 mmol) Bis(diphenylphosphino)ferrocenpalladium(II)chlorid und 3,72 g (26,9 mmol) Kaliumcarbonat wurden in 40 ml Dimethoxyethan 16 h bei 80°C unter Argon gerührt.

Zur Aufarbeitung wurde der Ansatz eingeengt und mittels Säulenchromatographie an Kieselgel (Laufmittel: Cyclohexan/Essigester) gereinigt.
Ausbeute: 778 mg (48% d.Th.)
HPLC-MS: LogP(HCOOH): 1,48; Masse (m/z): 180,9 (M+H)⁺;
¹H-NMR (d6-DMSO): 7,95 (m, 1H), 8,04 (m, 1H), 8,24 (m, 1H), 8,69 (m, 1H), 9,04 (m, 1H) ppm.

### Stufe 2: 6-[2-(5-Fluorpyridin-3-yl)-1,3-thiazol-5-yl]pyridin-2-carbonsäuremethylester

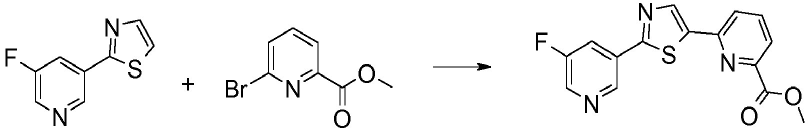

748 mg (4,15 mmol) des Fluorpyridyl-thiazols, 897 mg (4,15 mmol) des Brompyridins, 62 mg (0,13 mmol) Dihydrogen-dichloro-bis(di-t-butylphosphinito-kP)palladate(2-) (Dichlor{bis[di-tert-butyl(hydroxy)phosphoranyl]}palladium, POPd, Fa. CombiPhos, USA) und 1,147 g (8,3 mmol) Kaliumcarbonat wurden in 10 ml Dimethylformamid 16 h bei 120 °C unter Argon gerührt.

Zur Aufarbeitung wurde der Ansatz eingeengt und mittels Säulenchromatographie an Kieselgel (Laufmittel: Cyclohexan/Essigester) gereinigt.
Ausbeute: 615 mg (47% d.Th.)
HPLC-MS: LogP(HCOOH): 2,37; Masse (m/z): 316,0 (M+H)⁺;
¹H-NMR (d6-DMSO): 3,93 (s,3H), 8,03, (m, 1H), 8,14 (m, 1H), 8,36 (m,2H), 8,73 (m, 1H), 8,80 (m, 1H), 9,12 (m, 1H) ppm.

### Stufe 3: 6-[2-(5-Fluorpyridin-3-yl)-1,3-thiazol-5-yl]pyridin-2-carbonsäure

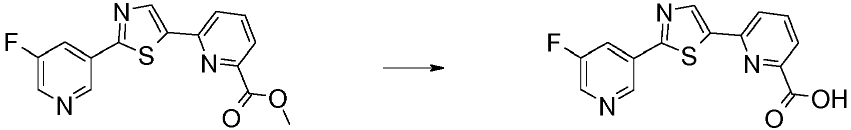

570 mg (1,81 mmol) des Pyridylcarbonsäure-methylesters wurden in einem Gemisch aus 25 ml Tetrahydrofuran und 8 ml Wasser gelöst, mit einer Lösung von 152 mg (3,62 mmol) Lithiumhydroxid-Monohydrat in 17 ml Wasser versetzt und 16 h bei Raumtemperatur gerührt.

Zur Aufarbeitung wurde das Gemisch eingeengt, zwischen Wasser und Methyl-t-butylether verteilt, die wässrige Phase mit IN Salzsäure angesäuert, der ausgefallene Feststoff abgesaugt und getrocknet.
Ausbeute: 556 mg (91% d.Th.)
HPLC-MS: LogP(HCOOH): 1,72; Masse (m/z): 302,1 (M+H)⁺;
¹H-NMR (d6-DMSO): 8,00 (m, 1H), 8,10 (m, 1H), 78,32 (m,2H), 8,72 (m, 1H), 8,78 (m, 1H), 9,10 (m, 1H), 13,5 (br) ppm.

### Stufe 4: 6-[2-(5-Fluorpyridin-3-yl)-1,3-thiazol-5-yl]-N-[(trifluormethyl)sulfonyl]pyridin-2-carboxamid

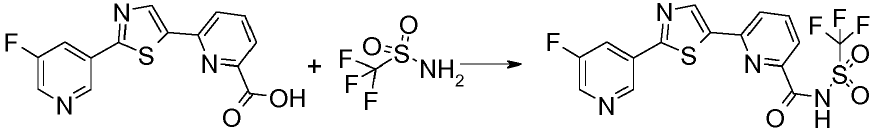

100 mg (0,33 mmol) der Pyridylcarbonsäure wurden in 5 ml Tetrahydrofuran vorgelegt, 81 mg (0,50 mmol) Carbonyldiimidazol zugesetzt und 1 h am Rückfluß gekocht. Nach Abkühlen auf Raumtemperatur wurden 74 mg (0,50 mmol) Trifluormethansulfonsäureamid zugesetzt, 10 min gerührt, anschließend 76 mg (0,50 mmol) Diazabicyclo-undecen (DBU) zugesetzt und 16 h bei Raumtemperatur gerührt.

Zur Aufarbeitung wurde das Gemisch eingeengt und mittels Säulenchromatographie an Kieselgel (Laufmittel: Dichlormethan / Methanol) gereinigt.
Ausbeute: 28 mg (20% d.Th.)
HPLC-MS: LogP(HCOOH): 1,86; Masse (m/z): 433,0 (M+H)⁺;
¹H-NMR (d6-DMSO): 7,60 (m, 1H), 7,88 (m, 1H), 7,96 (m, 1H), 8,12 (m, 1H), 8,30 (m, 1H), 8,70 (m, 1H), 8,89 (m, 1H), 9,10 (m, 1H) ppm.

### Beispiel C: 6-[5-(5-Fluorpyridin-3-yl)-1,3,4-thiadiazol-2-yl]-N-(methylsulfonyl)pyridin-2-carboxamid

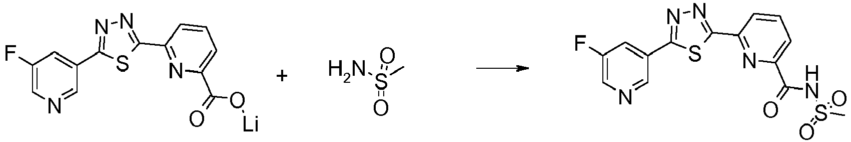

200 mg (0,64 mmol) des Lithiumsalzes und 1,01g (7,78 mmol) Diisopropyl-ethylamin wurden in 5 ml Acetonitril vorgelegt, 198 mg (0,77 mmol) Bis(2-oxo-3-oxazolidinyl) phosphinsäurechlorid (BOP-Cl) zugesetzt, 20 min gerührt, 185 mg (1,94 mmol) Methansulfonamid und 99 mg (0,64 mmol) Diazabicyclo-undecen (DBU) zugesetzt und 16 h bei Raumtemperatur gerührt.

Zur Aufarbeitung wurde das Gemisch eingeengt, zwischen Wasser und Essigester verteilt, die organische Phase getrocknet und eingeengt. Die weitere Reinigung erfolgte mittels Säulenchromatographie an Kieselgel (Laufmittel: Dichlormethan / Methanol) und anschließend an Kieselgel-RP18 (Laufmittel: Wasser/Acetonitril).
Ausbeute: 37 mg (15% d.Th.)
HPLC-MS: LogP(HCOOH): 1,79; Masse (m/z): 380,1 (M+H)⁺;
1H-NMR (d6-DMSO): 3,40 (s,3H), 8,22 (m, 1H), 8,30 (m, 1H), 8,48 (m, 1H), 8,59 (m, 1H), 8,82 (m, 1H), 9,18 (m, 1H), 12,1 (br) ppm.

### Beispiel D: 6-[1-(5-Fluorpyridin-3-yl)-1H-pyrazol-4-yl]-N-(methylsulfonyl)pyridin-2-carboxamid

### Stufe 1: 6-Brom-N-(methylsulfonyl)pyridin-2-carboxamid

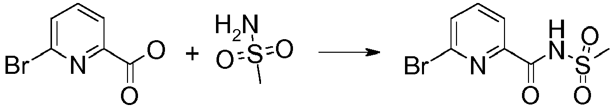

5,40 g (26,7 mmol) 6-Brom-2-pyridincarbonsäure wurden in 150 ml Tetrahydrofuran vorgelegt, 6,51 g (40,0 mmol) Carbonyldiimidazol zugesetzt und 1 h am Rückfluß gekocht. Nach Abkühlen auf Raumtemperatur wurden 3,81 g (40,0 mmol) Methansulfonsäureamid zugesetzt, 10 min gerührt, anschließend 6,10 g (40 mmol) Diazabicyclo-undecen (DBU) zugesetzt und 16 h bei Raumtemperatur gerührt.

Zur Aufarbeitung wurde das Gemisch eingeengt und mittels Säulenchromatographie an Kieselgel (Laufmittel: Dichlormethan / Methanol) gereinigt.
Ausbeute: 5,10g (69% d.Th.)
HPLC-MS: LogP(HCOOH): 1,21; Masse (m/z): 281,0 (M+H)⁺;
¹H-NMR (d6-DMSO): 3,37 (s,3H), 7,95 (m,2H), 8,06 (m, 1H) ppm.

### Stufe 2: -(Methylsulfonyl)-6-(1H-pyrazol-4-yl)pyridin-2-carboxamid

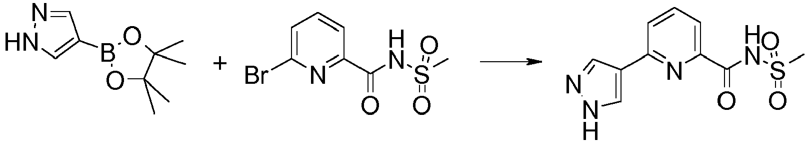

285 mg (1,47 mmol) des 4-Pyrazolboronesters, 410 mg (1,47 mmol) des Brompyridins, 32 mg (0,04 mmol) Bis(diphenylphosphino)ferrocenpalladium(II)chlorid und 467 (4,41 mmol) Kaliumcarbonat wurden in 10 ml Dimethoxyethan 16 h bei 80°C unter Argon gerührt.

Zur Aufarbeitung wurde der Ansatz eingeengt und mittels Säulenchromatographie an Kieselgel (Laufmittel: Cyclohexan/Essigester) gereinigt.
Ausbeute: 145 mg (37% d.Th.)
HPLC-MS: LogP(HCOOH): 0,91; Masse (m/z): 267,0 (M+H)⁺

### Stufe 3: 6-[1-(5-Fluorpyridin-3-yl)-1H-pyrazol-4-yl]-N-(methylsulfonyl)pyridin-2-carboxamid

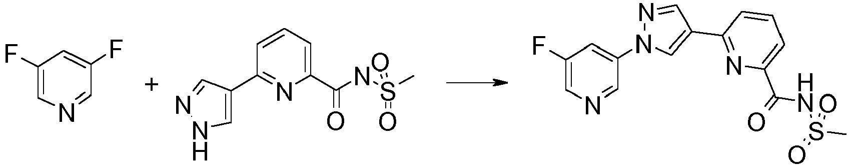

43 mg (0,37 mmol) 3,5-Difluorpyridin, 99 mg (0,37 mg) des Pyrazolyl-pyridins, 6 mg (0,01 mmol) Dihydrogen-dichloro-bis(di-t-butylphosphinito-kP)palladate(2-) (POPd, Fa. CombiPhos, USA) und 102 mg (0,74 mmol) Kaliumcarbonat wurden in 5 ml Dimethylformamid 12 h bei 120 °C unter Argon gerührt.

Zur Aufarbeitung wurde der Ansatz eingeengt und mittels Säulenchromatographie an Kieselgel (Laufmittel: Dichlormethan/Methanol) gereinigt.
Ausbeute: 35 mg (26% d.Th.)
HPLC-MS: LogP(HCOOH): 1,98; Masse (m/z): 362,1 (M+H)⁺;
¹H-NMR (d6-DMSO): 3,40 (s,3H), 7,98 (m, 1H), 8,08 (m,2H), 8,2 (m, 1H), 8,58 (m, 1H), 8,70 (m, 1H), 9,08 (m, 1H), 9,40 (m, 1H) ppm.

### Beispiel G: N-(Methylsulfonyl)-2-[1-(pyridin-3-yl)-1H-pyrazol-4-yl]-1,3-thiazol-4-carboxamid

### Stufe 1: 2-Brom-N-(methylsulfonyl)-1,3-thiazol-4-carboxamid

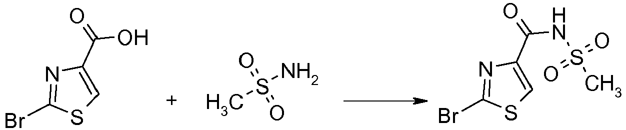

2-Brom-1,3-thiazol-4-carbonsäure (0,8 g, 3,85 mmol) wurde in Tetrahydrofuran (10 ml) vorgelegt. N,N'-carbonyldiimidazol (0,94 g, 5,77 mmol) wurde zugegeben und das Reaktionsgemisch 1 h unter Rückfluß erhitzt. Es wurde Methansulfonamid (0,55 g, 5,77 mmol) und nach 10 min 1,8-Diazabicyclo[5.4.0]undes-7-en (0,88 g, 5,77 mmol) zugegeben. Das Reaktionsgemisch wurde 16 h bei Raumtemperatur gerührt und anschließend das Lösemittel im Vakuum entfernt. Der Rückstand wurde in Wasser aufgenommen und mit Salzsäure angesäuert. Das ausgefallene Produkt wurde abgesaugt. Die wässrige Phase wurde mit Dichlormethan extrahiert, die organische Phase über Natriumsulfat getrocknet, filtriert und das Lösemittel im Vakuum entfernt. Es wurden insgesamt 1,0 g (89 % d.Th.) 2-Brom-N-(methylsulfonyl)-1,3-thiazol-4-carboxamid erhalten.
HPLC-MS: LogP(HCOOH): 0,83; Masse (m/z): 284,9 (M+H)⁺;
¹H-NMR (d6-DMSO): 3,33 (s ,3H), 8,61 (s, 1H), 12,00 (s, 1H)

### Stufe 2: N-(Methylsulfonyl)-2-[1-(pyridin-3-yl)-1H-pyrazol-4-yl]-1,3-thiazol-4-carboxamid

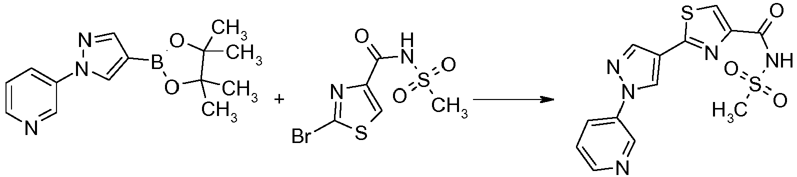

Unter Argon wurden 3-[4-(4,4,5,5-tetramethyl-1,3,2-dioxaborolan-2-yl)-1H-pyrazol-1-yl]pyridin (0,1 g, 0,37 mmol), 2-Brom-N-(methylsulfonyl)-1,3-thiazol-4-carboxamid (0,105 g, 0,37 mmol) und Tetrakis(triphenylphosphine)palladium (0,013 g, 0,011 mmol) zu einer mittels Argon entgasten Mischung aus Natriumcarbonatlösung in Wasser (1,5 ml, 2 M/L) und Acetonitril (3,8 ml) gegeben. Das Reaktionsgemisch wurde 16 h bei 70 °C gerührt. Nach Abkühlen wurde das Reaktionsgemisch auf Wasser gegossen und mit Dichlormethan extrahiert. Die organische Phase wurde über Natriumsulfat getrocknet und das Lösemittel im Vakuum entfernt. Der Rückstand wurde anschließend mit Diethylether verrührt und abgesaugt. Es wurden 0,23 g (97 % d.Th.) 6-[1-(Pyridin-3-yl)-1H-pyrazol-4-yl]pyridin-2-sulfonamid erhalten.
HPLC-MS: LogP(HCOOH): 1,25; Masse (m/z): 350,1 (M+H)⁺
¹H-NMR (d6-DMSO): 3,38(S,3H), 7,48-7,49(m,1H), 8,32-8,35(m,1H), 8,45(s,1H), 8,56(s,1H), 8,59-8,60(m,1H), 9,19-9,20(m,1H), 9,34(s,1H) ppm

### Beispiel H: 4-[1-(5-Fluorpyridin-3-yl)-1H-pyrazol-4-yl]-N-(methylsulfonyl)-1,3-thiazol-2-carboxamid

### Stufe 1: 4-Brom-N-(methylsulfonyl)-1,3-thiazol-2-carboxamid

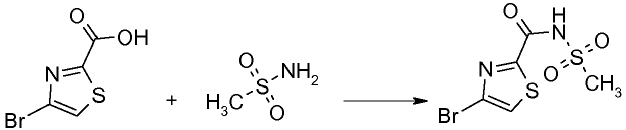

4-Brom-1,3-thiazol-2-carbonsäure (1,0 g, 4,8 mmol) wurde in Tetrahydrofuran (10 ml) vorgelegt. N,N'-carbonyldiimidazol (1,17 g, 7,2 mmol) wurde zugegeben und das Reaktionsgemisch 1 h unter Rückfluß erhitzt. Es wurde Methansulfonamid (0,69 g, 7,2 mmol) und nach 10 min 1,8-Diazabicyclo[5.4.0]undes-7-en (1,10 g, 7,2 mmol) zugegeben. Das Reaktionsgemisch wurde 16 h bei Raumtemperatur gerührt und anschließend das Lösemittel im Vakuum entfernt. Der Rückstand wurde in Wasser aufgenommen und mit Salzsäure angesäuert. Das ausgefallene Produkt wurde abgesaugt. Es wurden 1,18 g (84 % d.Th.) 4-Brom-N-(methylsulfonyl)-1,3-thiazol-2-carboxamid erhalten.
HPLC-MS: LogP(HCOOH): 0,63; Masse (m/z): 284,9 (M+H)⁺;
¹H-NMR (d6-DMSO): 3,31 (s ,3H), 8,31 (s, 1H)

### Stufe 2: 4-[1-(5-Fluorpyridin-3-yl)-1H-pyrazol-4-yl]-N-(methylsulfonyl)-1,3-thiazol-2-carboxamid

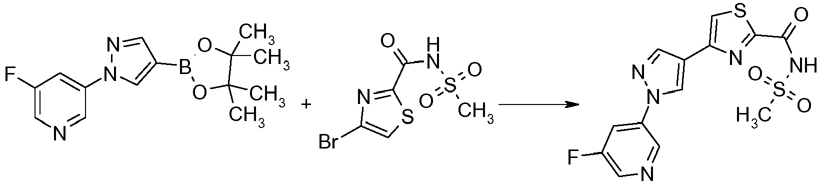

Unter Argon wurden 3-Fluor-5-[4-(4,4,5,5-tetramethyl-1,3,2-dioxaborolan-2-yl)-1H-pyrazol-1-yl]pyridin (0,2 g, 0,69 mmol, hergestellt nach den in WO 2011/045224 beschriebenen Methoden), 2-Brom-N-(methylsulfonyl)-1,3-thiazol-4-carboxamid (0,2 g, 0,69 mmol) und Tetrakis(triphenylphosphine)palladium (0,024 g, 0,021 mmol) zu einer mittels Argon entgasten Mischung aus Natriumcarbonatlösung in Wasser (2,8 ml, 2 M/L) und Acetonitril (9,4 ml) gegeben. Das Reaktionsgemisch wurde 16 h bei 70 °C gerührt. Nach dem Abkühlen wurde das Reaktionsgemisch auf Wasser gegossen und mit Dichlormethan extrahiert. Die wässrige Phase wurde mit Salzsäure angesäuert und der ausgefallene Feststoff abgesaugt. Das Rohprodukt wurde mit Ethylacetat/2-Propanol an Kieselgel chromatographiert. Es wurden 0,21 g (8 % d.Th) 4-[1-(5-Fluorpyridin-3-yl)-1H-pyrazol-4-yl]-N-(methylsulfonyl)-1,3-thiazol-2-carboxamid erhalten.
HPLC-MS: LogP(HCOOH): 1,68; Masse (m/z): 368,1 (M+H)⁺
¹H-NMR (d6-DMSO): 2,99 (s, 3H), 7,96 (s, 1H), 8,33 (s, 1H), 8,34 (s, 1H), 8,55-8,57 (m, 1H), 9,10 (s, 1H), 9,12 (s, 1H) ppm

### Beispiel I: 3-[2-(5-Fluorpyridin-3-yl)-4-methyl-1,3-thiazol-5-yl]-1-methyl-N-(methylsulfonyl)-1H-pyrazol-5-carboxamid

### Stufe 1: Ethyl-4-[2-(5-fluorpyridin-3-yl)-4-methyl-1,3-thiazol-5-yl]-2,4-dioxobutanoat

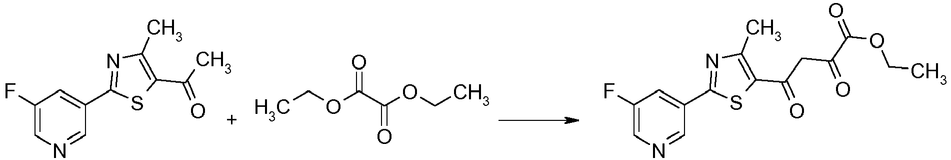

Unter Argon wurde Lithium Bis(trimethylsilyl)amid (21 ml, 1 M/L in Tetrahydrofuran) in Tetrahydrofuran (150 ml) vorgelegt. Bei -78 °C wurde 1-[2-(5-Fluorpyridin-3-yl)-4-methyl-1,3-thiazol-5-yl]ethanon (5,0 g, 21 mmol, hergestellt analog zu Biorg.&Med.Chem.Lett 1056(2007) und 2828(2010)) gelöst in Diethylether langsam zugetropft und das Reaktionsgemisch 2 h gerührt. Oxalsäurediethylester (3,1 g, 21 mmol) gelöst in Ether wurde zugetropft und das Reaktionsgemisch 16 h bei Raumtemperatur gerührt. Anschließend wurde Kaliumhydrogensulfatlösung (5 %ig) zugegeben und mehrfach mit Ethylacetat extrahiert. Die vereinigten organischen Phasen wurden getrocknet und das Lösemittel im Vakuum entfernt. Es wurden 7,1 g (99 % d.Th) Ethyl-4-[2-(5-fluorpyridin-3-yl)-4-methyl-1,3-thiazol-5-yl]-2,4-dioxobutanoat erhalten.
HPLC-MS: LogP(HCOOH): 3,37; Masse (m/z): 337,1 (M+H)⁺
¹H-NMR (d6-DMSO): 1,31 (t, 3H), 2,79 (s, 3H), 4,31-4,32 (m, 2H), 6,71 (bs, 2H), 8,33 (d, 1H), 8,77 (s, 1 H), 9,10 (s, 1 H) ppm

### Stufe 2: Ethyl-3-[2-(5-fluorpyridin-3-yl)-4-methyl-1,3-thiazol-5-yl]-1-methyl-1H-pyrazol-5-carboxylat

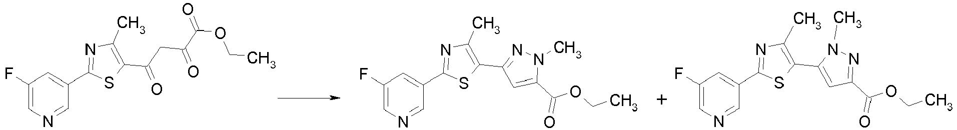

Ethyl-4-[2-(5-fluorpyridin-3-yl)-4-methyl-1,3-thiazol-5-yl]-2,4-dioxobutanoat (6,9 g, 20,5 mmol) und Methylhydrazin (0,65 g, 20,5 mmol) wurden in Ethanol (250 ml) 2h unter Rückfluß erhitzt. Das entstandene Produkt wurde abgesaugt und das Lösemittel des Filtrats unter Vakuum entfernt. Der Rückstand wurde an Kieselgel chromatographiert (Laufmittel Cyclohexan/Essigester). Es wurden insgesamt 1,24 g (17 % d.Th) Ethyl-3-[2-(5-fluorpyridin-3-yl)-4-methyl-1,3-thiazol-5-yl]-1-methyl-1H-pyrazol-5-carboxylat erhalten. Als Nebenprodukt wurden 4,8 g (67 % d.Th.) Ethyl-5-[2-(5-fluorpyridin-3-yl)-4-methyl-1,3-thiazol-5-yl]-1-methyl-1H-pyrazol-3-carboxylat erhalten.
HPLC-MS: LogP(HCOOH): 3,70; Masse (m/z): 347,0 (M+H)⁺
¹H-NMR (d6-DMSO): 1,35 (t, 3H), 2,63 (s, 3H), 4,15 (s, 3H), 4,35 (q, 2H), 7,17 (s, 1H), 8,19-8,22 (m, 1H), 8,68-8,69 (m, 1H), 9,00-9,01 (m, 1H) ppm

### Stufe 3: 3-[2-(5-Fluorpyridin-3-yl)-4-methyl-1,3-thiazol-5-yl]-1-methyl-1H-pyrazol-5-carbonsäure

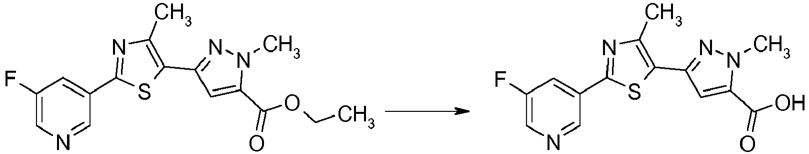

Ethyl-3-[2-(5-fluorpyridin-3-yl)-4-methyl-1,3-thiazol-5-yl]-1-methyl-1H-pyrazol-5-carboxylat (1,1 g, 3,1 mmol) wurde in Tetrahydrofuran (200 ml) und Wasser (100 ml) gelöst und Lithiumhydroxid Monohydrat (0,26 g, 6,2 mmol) gelöst in Wasser (100 ml) zugegeben. Das Reaktionsgemisch wurde 16 h bei Raumtemperatur gerührt, anschließend mit Salzsäure neutralisiert und das Lösemittel im Vakuum entfernt. Der ausgefallene Feststoff wurde abgesaugt. Es wurden 0,96 g (94 % d.Th) 3-[2-(5-Fluorpyridin-3-yl)-4-methyl-1,3-thiazol-5-yl]-1-methyl-1H-pyrazol-5-carbonsäure erhalten.
HPLC-MS: LogP(HCOOH): 2,05; Masse (m/z): 319,0 (M+H)⁺
¹H-NMR (d6-DMSO): 2,62 (s, 3H), 4,14 (s, 3H), 7,13 (s, 1H), 8,18-8,22 (m, 1H), 8,68-8,69 (m, 1H), 9,00-9,01 (m, 1H) ppm

### Stufe 4: 3-[2-(5-Fluorpyridin-3-yl)-4-methyl-1,3-thiazol-5-yl]-1-methyl-N-(methylsulfonyl)-1H-pyrazol-5-carboxamid

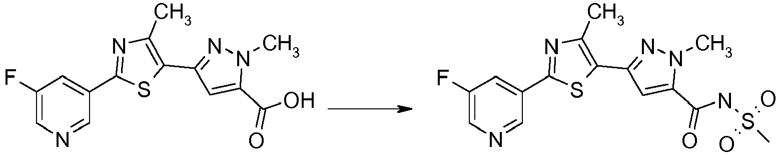

3-[2-(5-Fluorpyridin-3-yl)-4-methyl-1,3-thiazol-5-yl]-1-methyl-1H-pyrazol-5-carbonsäure (0,08 g, 0,25 mmol) und Methansulfonamid (0,024 g, 0,25 mmol) wurden unter Eiskühlung in Dichlormethan (10 ml) vorgelegt. 4-Dimethylaminopyridin (0,006 g, 0,05 mmol) und N-(3-Dimethylaminopropyl)-N'-ethylcarbodiimid Hydrochlorid (0,053 g, 0,275 mmol) wurden zugegeben. Das Reaktionsgemisch wurde 2 h bei 0 °C und anschließend 16 h bei Raumtemperatur gerührt. Das Lösemittel wurde im Vakuum entfernt und der Rückstand an Kieselgel chromatographiert (Laufmittel Dichlormethan / Essigester). Es wurden 0,074g (72% d.Th) 3-[2-(5-Fluorpyridin-3-yl)-4-methyl-1,3-thiazol-5-yl]-1-methyl-N-(methylsulfonyl)-1H-pyrazol-5-carboxamid erhalten.
HPLC-MS: LogP(HCOOH): 2,04; Masse (m/z): 396,0 (M+H)⁺
¹H-NMR (d6-DMSO): 2.55 (s,3H), 3.40 (s,3H), 4.12 (s,3H), 7.68 (m,1H), 8.12 (m,1H), 8.69 (m,1H), 9.01 (m,1H) ppm

Analog bzw. gemäß den oben beschriebenen Herstellverfahren wurden die folgenden Verbindungen der Formel (I) erhalten:

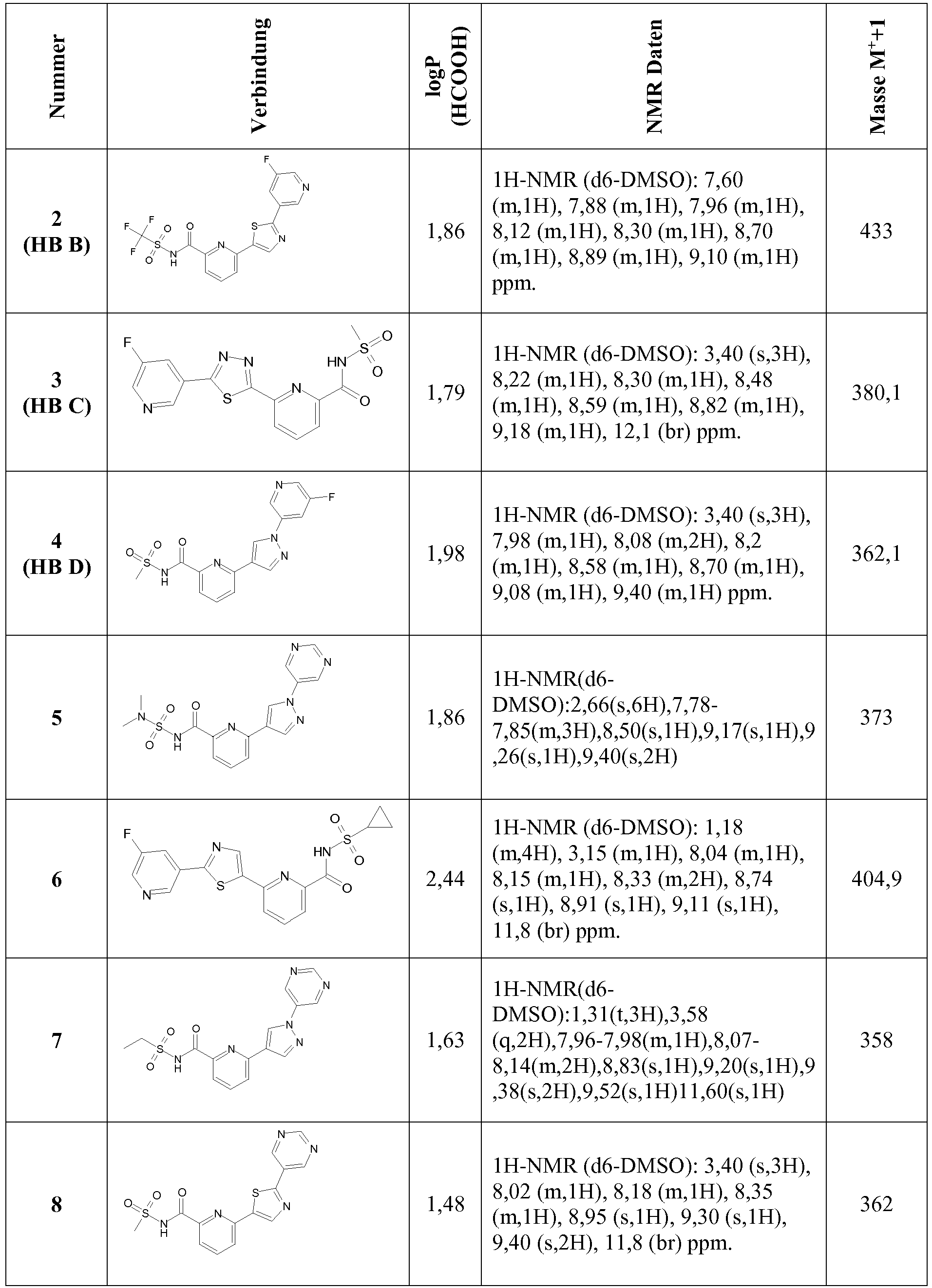

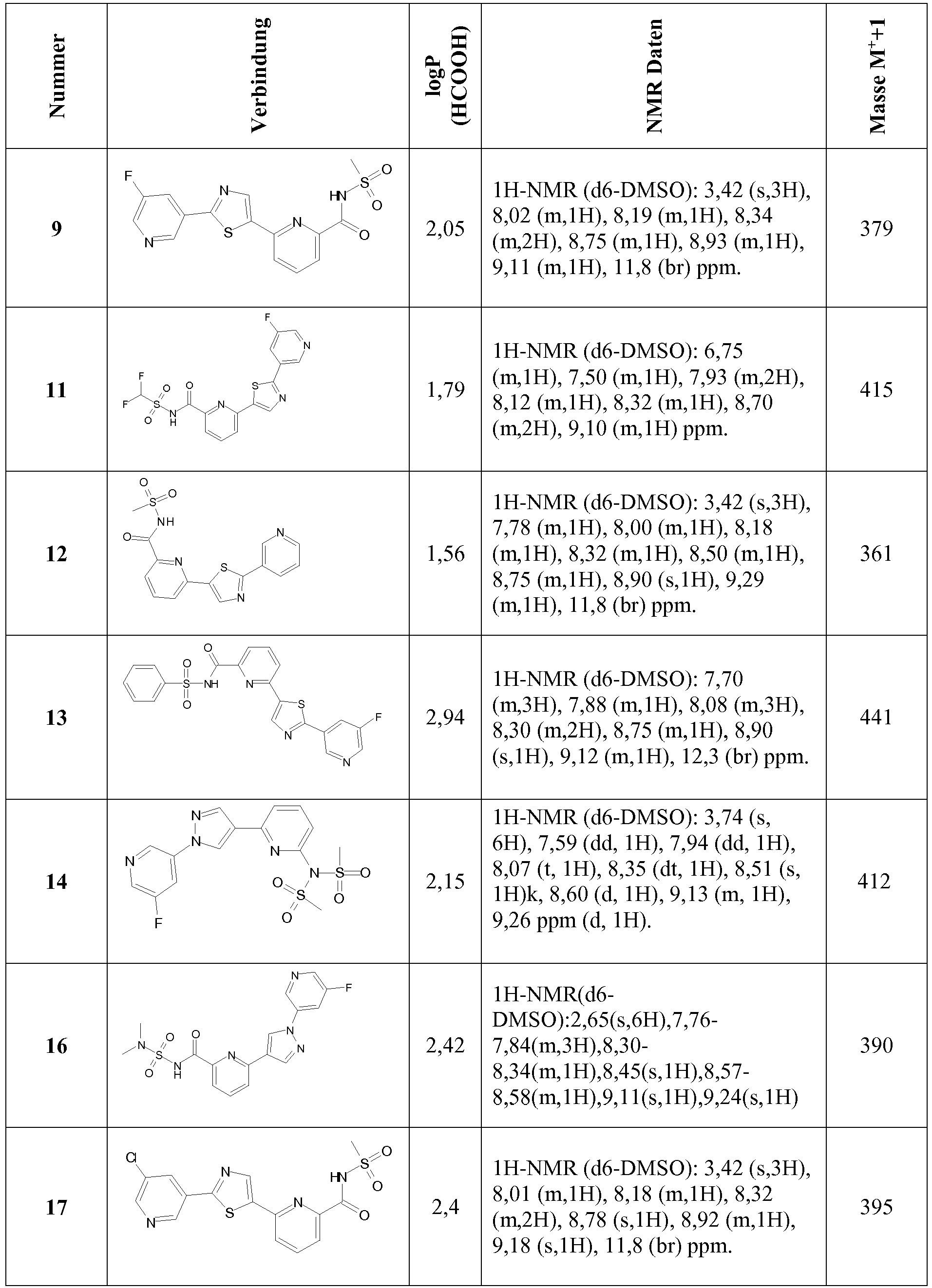

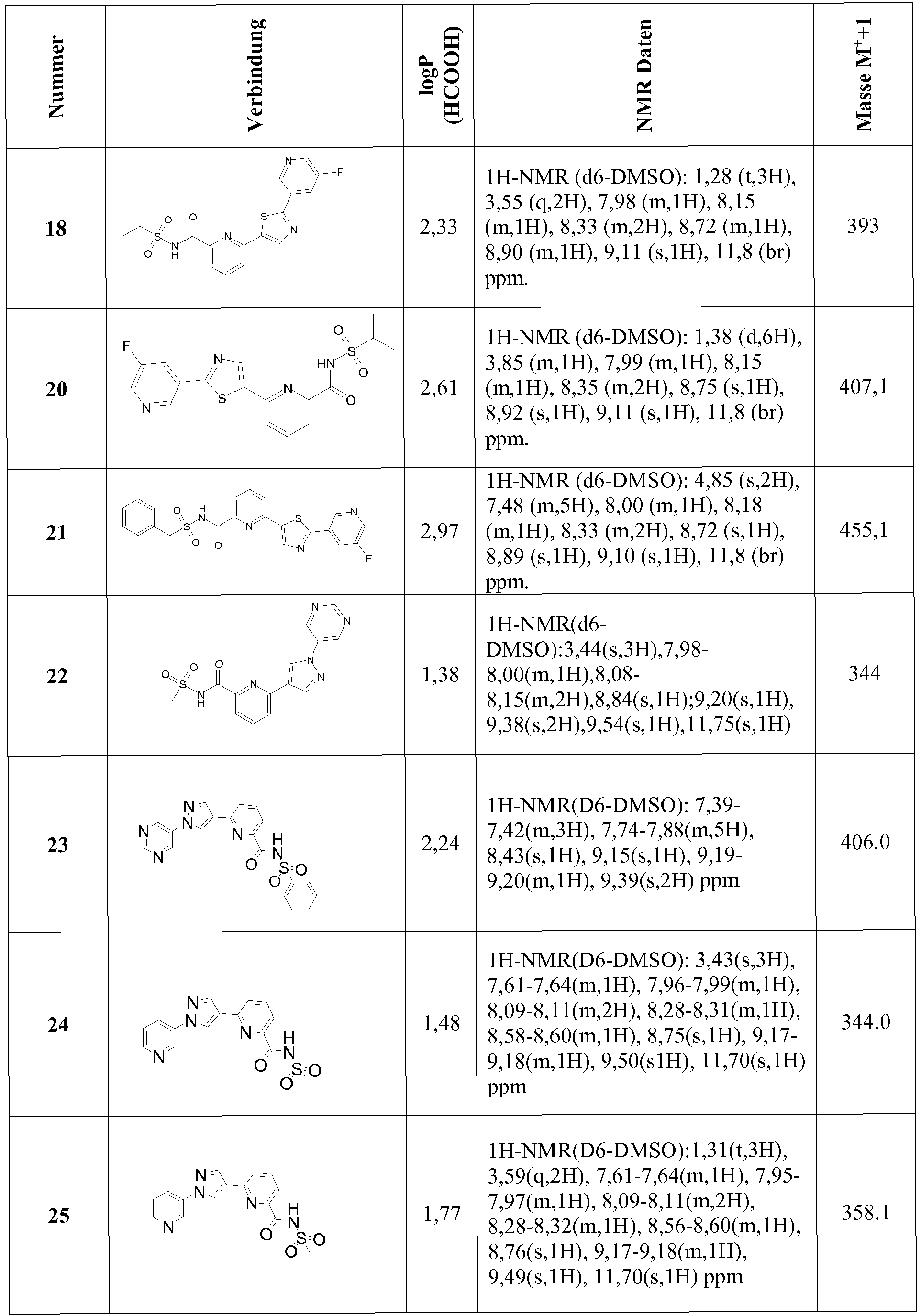

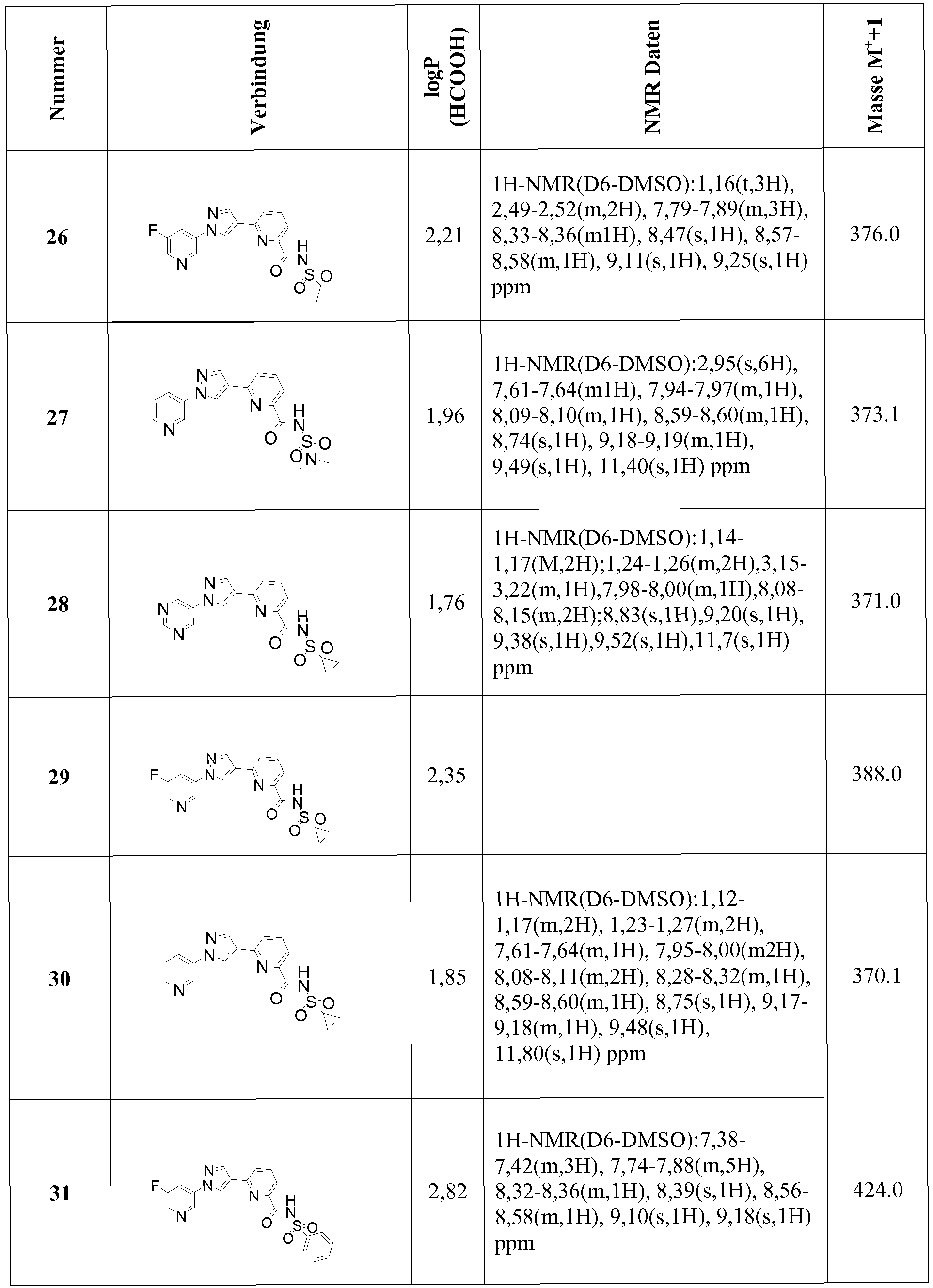

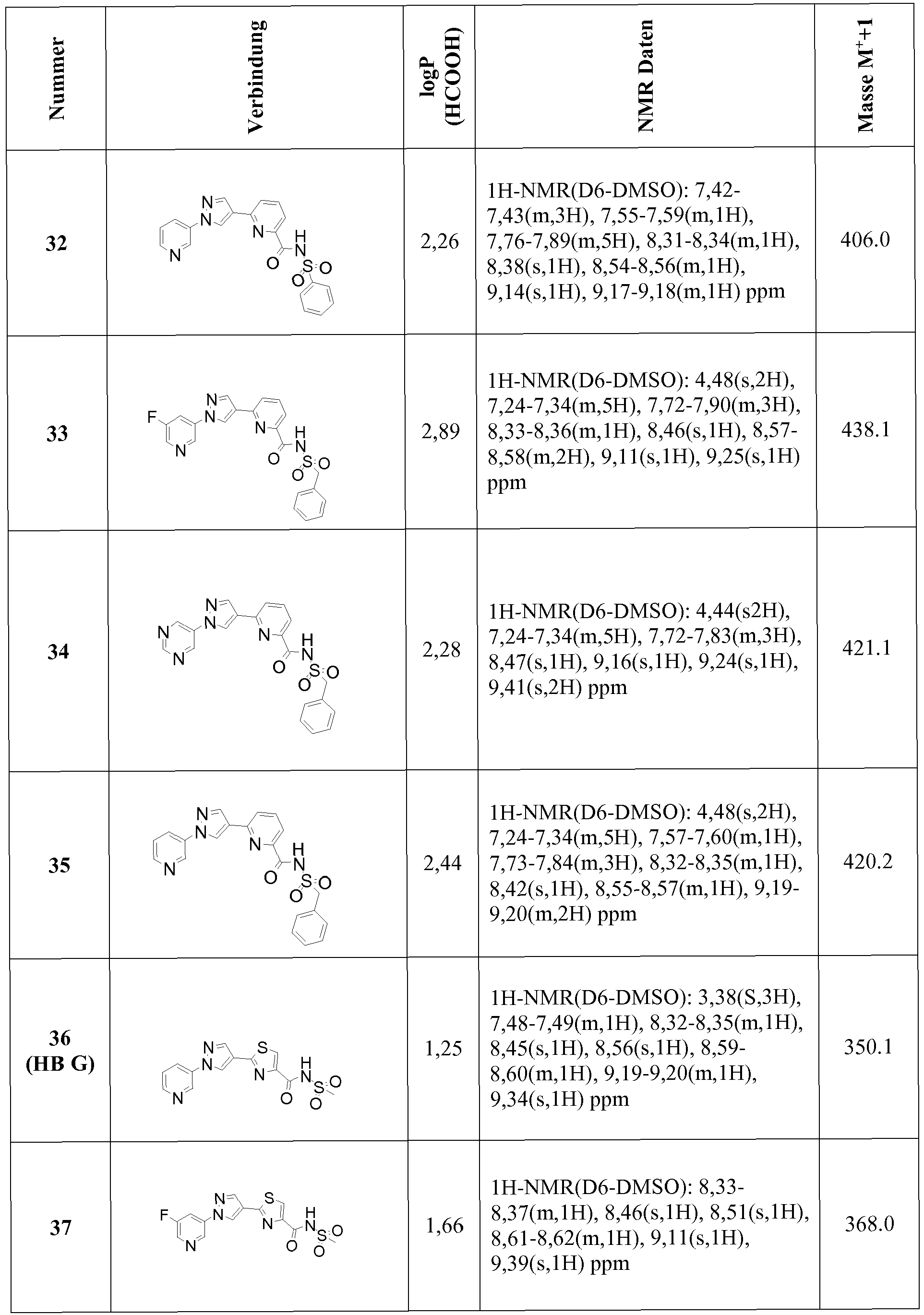

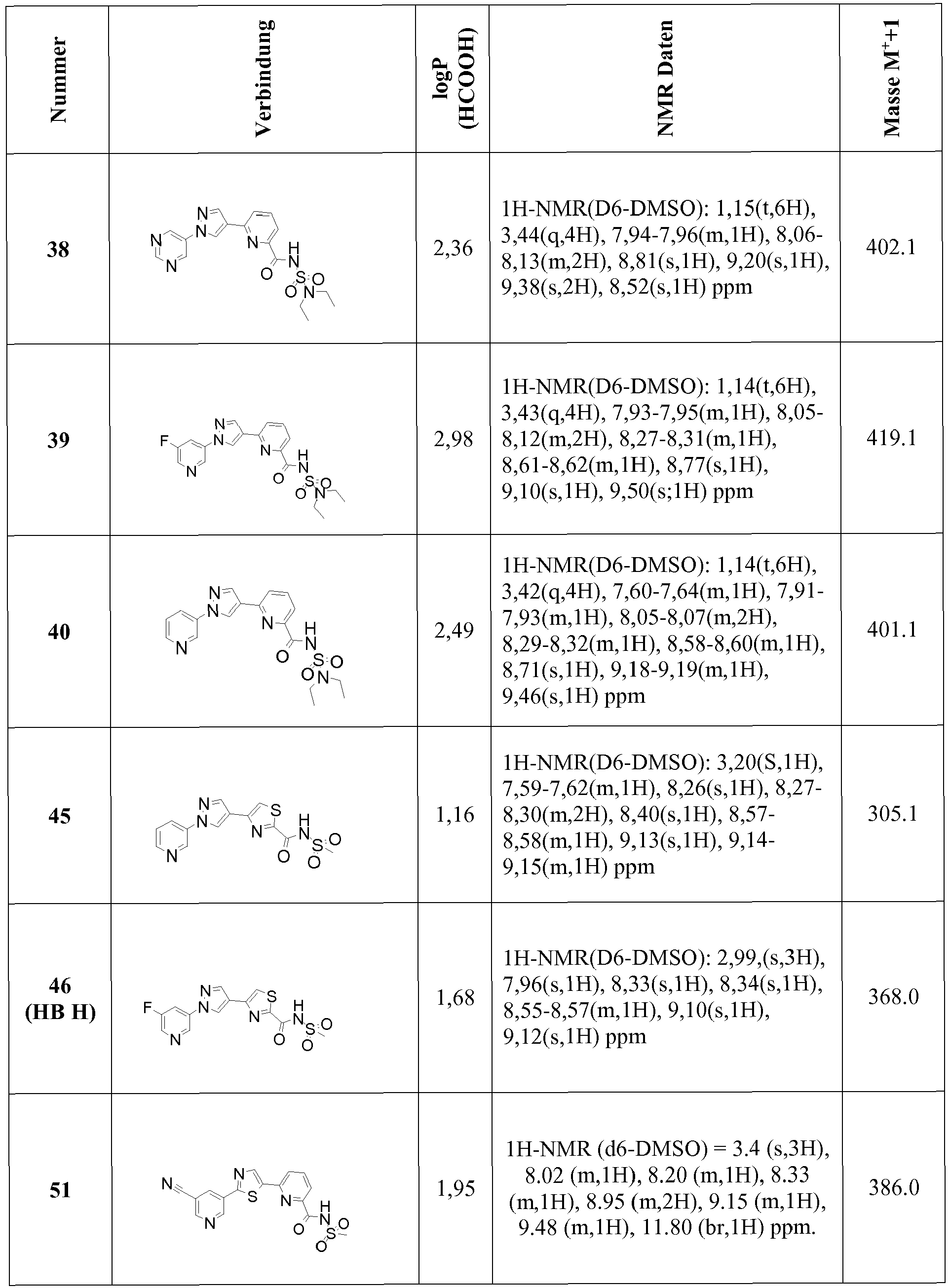

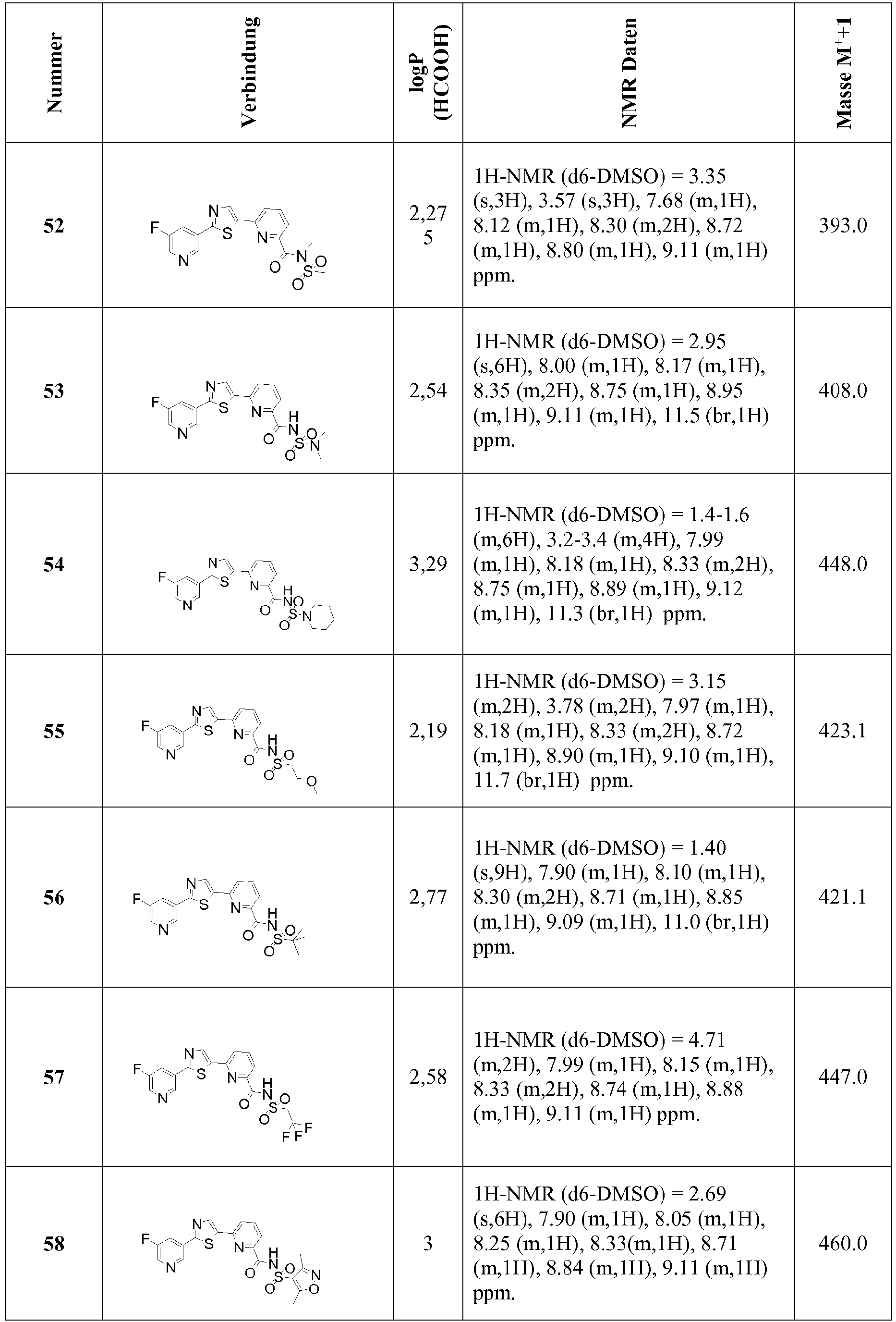

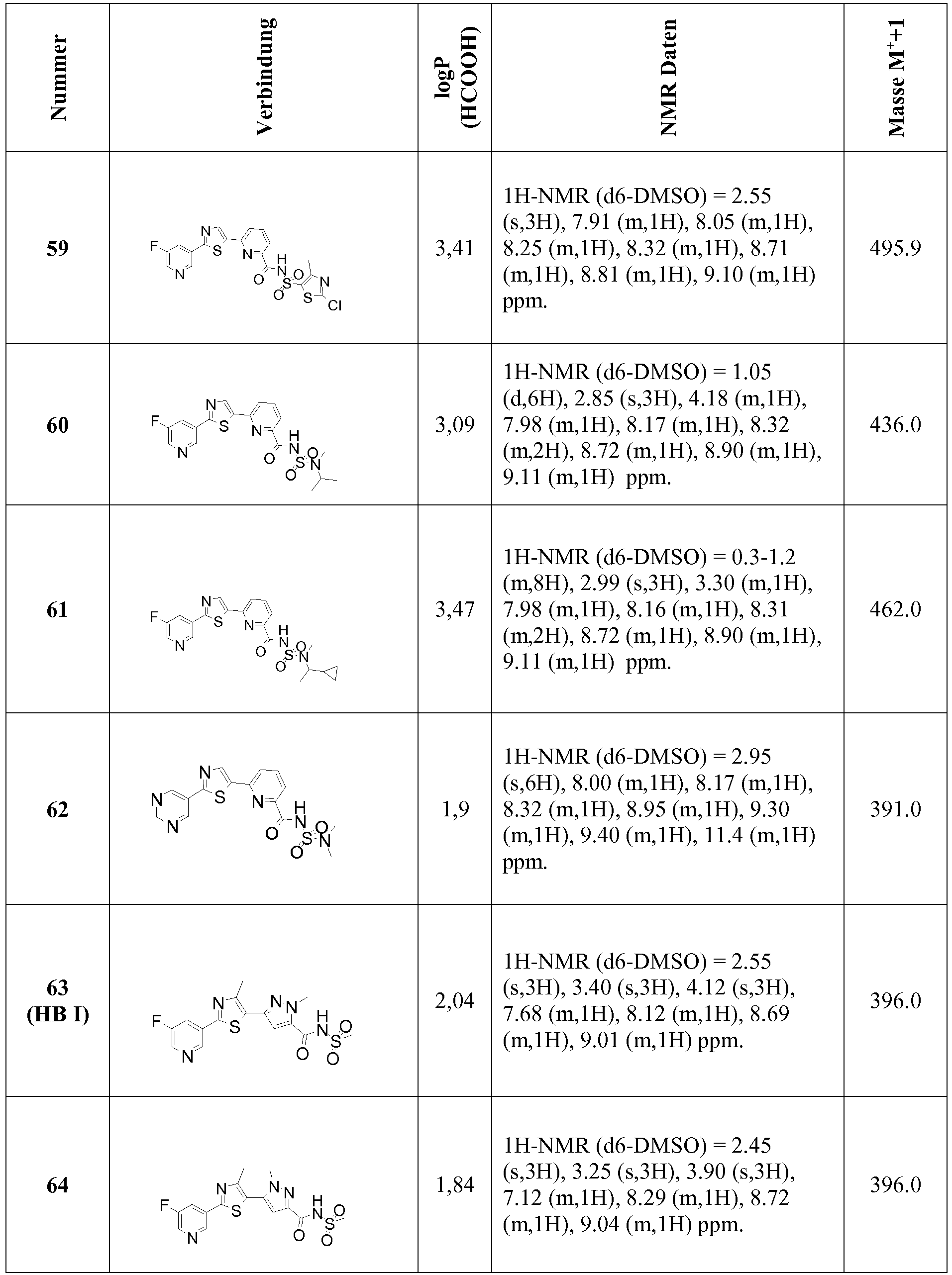

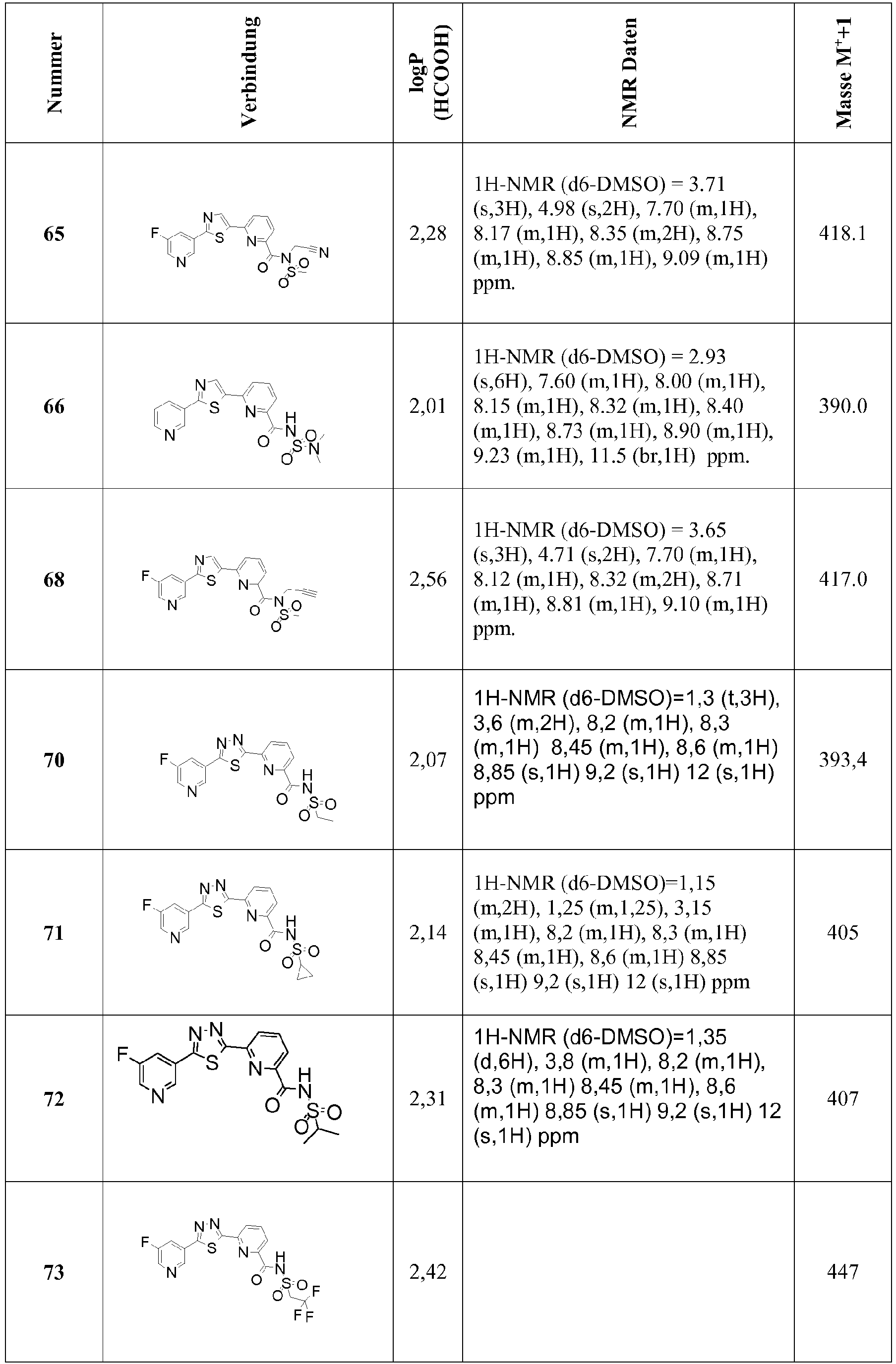

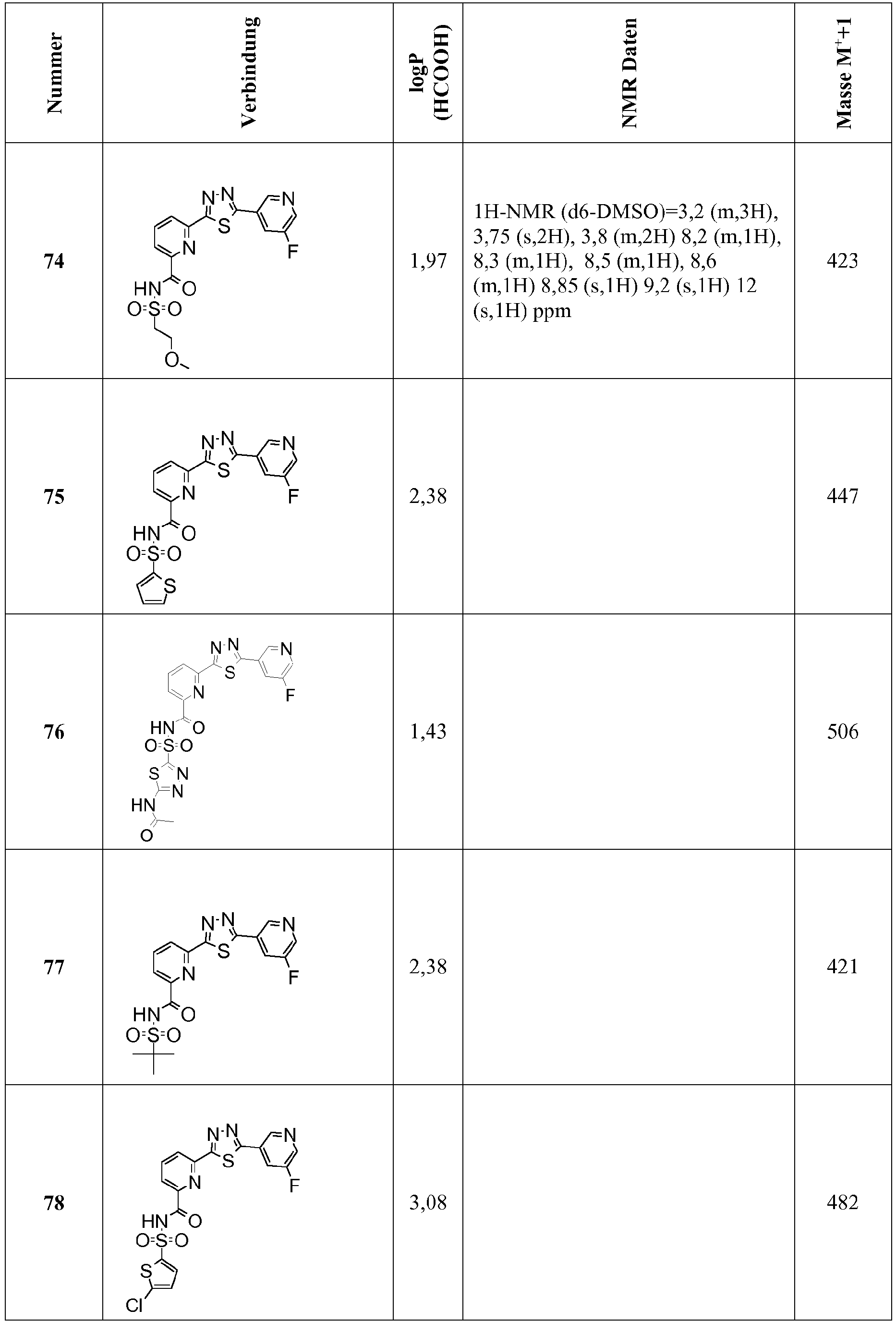

### HB = Herstellungsbeispiel

Gemäß den oben beschriebenen Herstellverfahren wurden die folgenden Zwischenprodukte der Formel (XII) erhalten:

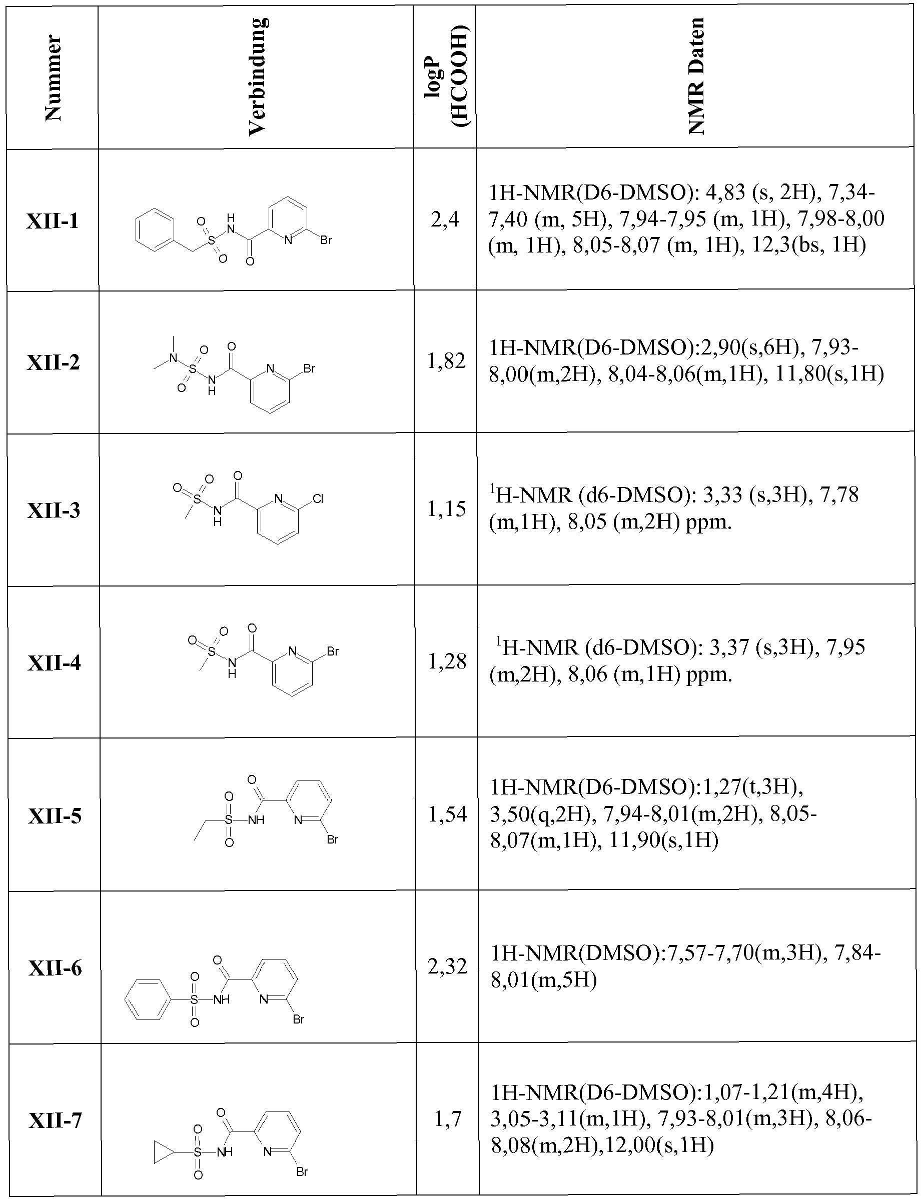

Gemäß den oben beschriebenen Herstellverfahren wurden die folgenden Zwischenprodukte der Formel (XV) erhalten:

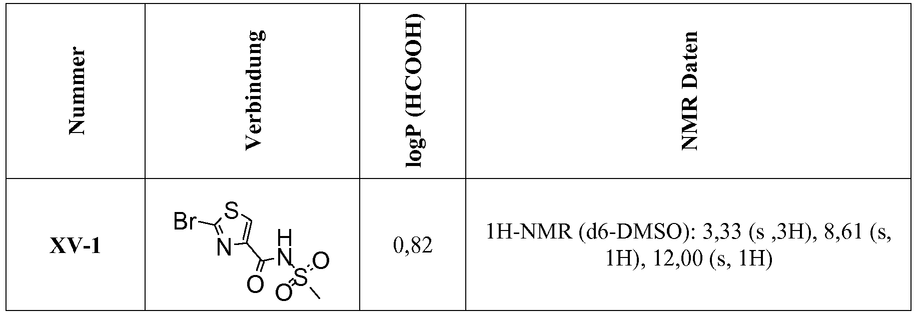

Gemäß den oben beschriebenen Herstellverfahren wurden die folgenden Zwischenprodukte der Formel (XVI) erhalten:

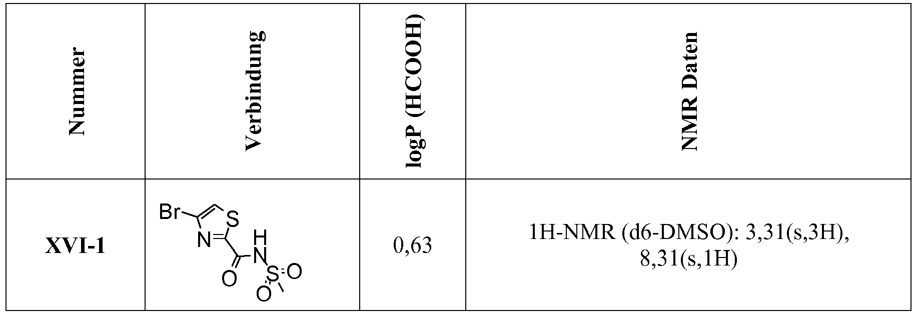

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### 2) Messung der NMR-Spektren

Die NMR-Spektren wurden mit einem Bruker Avance 400, ausgestattet mit einem Durchflussprobenkopf (60 µl Volumen), bestimmt. Als Lösungsmittel wurden CD₃CN oder d₆-DMSO verwendet, wobei als Referenz Tetramethylsilan (0.00 ppm) eingesetzt wurde. In Einzelfällen wurden die NMR Spektren mit einem Bruker Avance II 600 bestimmt. Als Lösungsmittel wurden CD₃CN oder d₆-DMSO verwendet, wobei als Referenz Tetramethylsilan (0.00 ppm) eingesetzt wurde.

Die Aufstpaltung der Signale wurde wie folgt beschrieben: s (Singulett), d (Duplett), t (Triplett), q (Quartett), quin (Quintett), m (Multiplett).

### Biologische Beispiele

**Myzus-Test (Spritzbehandlung)**

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*)*,* die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigten z. B. die folgenden Verbindungen der Herstellungsbeispiele Wirkung von 80 % bei einer Aufwandmenge von 500 g/ha: 1, 10, 15, 21, 28, 62, 63

Bei diesem Test zeigten z. B. die folgenden Verbindungen der Herstellungsbeispiele Wirkung von 90 % bei einer Aufwandmenge von 500 g/ha: 5, 14, 23, 32, 34, 37, 38, 45, 47, 48, 65, 74

Bei diesem Test zeigten z. B. die folgenden Verbindungen der Herstellungsbeispiele Wirkung von 100 % bei einer Aufwandmenge von 500 g/ha: 2, 3, 4, 6, 7, 8, 9, 11, 12, 13, 16, 17, 18, 20, 22, 24, 25, 26, 27, 29, 30, 31, 33, 35, 36, 39, 40, 46, 51, 52, 53, 54, 55, 56, 57, 58, 59, 60, 61, 64, 66, 68, 70, 71, 72, 73, 75, 76, 77, 78.

**Tetranychus - test, OP-resistent (Spritzbehandlung)**

| | | |
|---|---|---|
| Lösungsmittel: | 78,0 | Gewichtsteile Aceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt. Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigte z. B. die folgende Verbindung der Herstellungsbeispiele eine Wirkung von 100 % bei einer Aufwandmenge von 500 g/ha: 5.

### Meloidogyne incognita- Test

### Lösungsmittel: 80,0 Gewichtsteile Aceton

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, Meloidogyne incognita Ei- Larven- Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigten z. B. die folgenden Verbindungen der Herstellungsbeispiele eine Wirkung von 100 % bei einer Aufwandmenge von 20 ppm: 56, 70, 75, 78.

## Patentansprüche

1. Verbindungen der Formel (I), 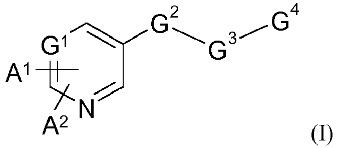 worin
A¹ und A² unabhängig voneinander für Wasserstoff, Halogen, Cyano, Nitro, C₁-C₆-Alkyl, C₃-C₆-Cycloalkyl oder C₁-C₆-Alkoxy stehen,
G¹ für N oder C-A¹ steht,
G² für einen Rest aus der Reihe 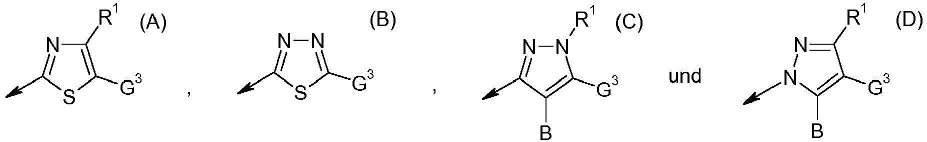 steht, worin der Pfeil die Bindung zum benachbarten Ring markiert,
R¹ im Falle der Heterocyclen (A) und (D) für Wasserstoff, Halogen, Cyano, C₁-C₆-Alkyl, C₁-C₆-Alkoxy oder C₁-C₆-Haloalkyl steht,
R¹ im Falle von Heterocyclus (C) für Wasserstoff, C₁-C₆-Alkyl oder C₁-C₆-Haloalkyl steht,
B für Wasserstoff, Halogen, Cyano, Nitro, C₁-C₆-Alkyl, C₁-C₆-Haloalkyl, C₁-C₆-Alkoxy oder C₃-C₆-Cycloalkyl steht,
G³ für jeweils gegebenenfalls durch Halogen, Cyano, Nitro, Amino, C₁-C₆-Alkylamino, Di-(C₁-C₆)-alkylamino, C₁-C₆-Alkyl, C₃-C₆-Cycloalkyl, C₁-C₆-Halogenalkyl, Hydroxy, C₁-C₆-Alkoxy, C₁-C₆-Halogenalkoxy, C₁-C₆-Alkylthio, C₁-C₆-Alkylcarbonyl, C₁-C₆-Alkoxycarbonyl, C₂-C₆-Alkenyl oder C₂-C₆-Alkinyl substituiertes 5-gliedriges Heteroaryl oder 6-gliedriges Heteroaryl steht,
G⁴ für einen Rest aus der Reihe 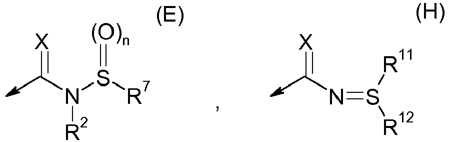 steht,
X für Sauerstoff oder Schwefel steht,
n für 1 oder 2 steht,
R² für einen Rest aus der Reihe Wasserstoff, C₁-C₆-Alkyl, C₁-C₆-Halogenalkyl, Cyano-C₁-C₆-alkyl, C₁-C₆-Alkoxy, C₁-C₆-Halogenalkoxy, C₂-C₆-Alkenyl, C₂-C₆-Alkinyl, C₁-C₆-Alkoxy-C₁-C₆-alkyl, jeweils gegebenenfalls durch Halogen substituiertes C₁-C₆-Alkylcarbonyl und C₁-C₆-Alkylsulfonyl, gegebenenfalls durch Halogen substituiertes C₁-C₆-Alkoxycarbonyl, gegebenenfalls durch Halogen, C₁-C₆-Alkyl, C₁-C₆-Alkoxy, C₁-C₆-Halogenalkyl und Cyano substituiertes C₃-C₆-Cycloalkylcarbonyl, oder für ein ein- oder zweiwertiges Metallion oder ein gegebenenfalls durch C₁-C₆-Alkyl oder Aryl-C₁-C₆-alkyl substituiertes Ammonium-Ion steht,
R⁷ für einen Rest aus der Reihe jeweils gegebenenfalls durch Halogen, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl, C₃-C₆-Cycloalkyl-C₁-C₆-alkyl und C₃-C₆-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Aryl, Heteroaryl, Aryl-C₁-C₆-alkyl und Heteroaryl-C₁-C₆-alkyl oder für NR'R", worin R' und R" unabhängig voneinander jeweils für einen Rest aus der Reihe Wasserstoff, C₁-C₆-Alkyl, C₁-C₆-Haloalkyl, C₃-C₆-Cycloalkyl, C₃-C₆-Cycloalkyl-C₁-C₆-alkyl, C₁-C₆-Alkoxy, C₁-C₆-Alkylcarbonyl und C₁-C₆-Alkoxylcarbonyl steht,
R² und R⁷ auchzusammen mit der N-S(O)ₙ Gruppe, an die sie gebunden sind, einen gesättigten oder ungesättigten und gegebenenfalls durch Halogen, C₁-C₆-Alkyl, C₁-C₆-Halogenalkyl, C₁-C₆-Alkoxy, C₁-C₆-Halogenalkoxy substituierten 5- bis 7-gliedrigen Ring bilden können, der ein oder mehrere weitere Heteroatome aus der Reihe Schwefel, Sauerstoff (wobei Sauerstoffatome nicht unmittelbar benachbart sein dürfen) und Stickstoff und/oder mindestens eine Carbonylgruppe enthalten kann,
R¹¹ und R¹² unabhängig voneinander für einen jeweils gegebenenfalls durch Halogen, C₁-C₆-Alkyl, C₁-C₆-Halogenalkyl, C₁-C₆-Alkoxy, C₁-C₆-Halogenalkoxy substituierten Rest aus der Reihe C₁-C₆-Alkyl, C₂-C₆-Alkenyl, C₂-C₆-Alkinyl, Phenyl und Phenyl-C₁-C₆-alkyl stehen,
und wobei
Halogen ausgewählt ist aus der Reihe Fluor, Chlor, Brom und Iod, Aryl (auch als Teil einer größeren Einheit, wie beispielsweise Arylalkyl) ausgewählt ist aus der Reihe Phenyl, Naphthyl, Anthryl, Phenanthrenyl, Hetaryl (gleichbedeutend mit Heteroaryl, auch als Teil einer größeren Einheit, wie beispielsweise Hetarylalkyl) ausgewählt ist aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl, sowie Salze und N-Oxide der Verbindungen der Formel (I).

2. Verbindungen der Formel (I) gemäß Anspruch 1, worin
G³ für jeweils gegebenenfalls durch Halogen, Cyano, Nitro, Amino, C₁-C₆-Alkylamino, Di-(C₁-C₆)-alkylamino, C₁-C₆-Alkyl, C₃-C₆-Cycloalkyl, C₁-C₆-Halogenalkyl, Hydroxy, C₁-C₆-Alkoxy, C₁-C₆-Halogenalkoxy, C₁-C₆-Alkylthio, C₁-C₆-Alkylcarbonyl, C₁-C₆-Alkoxycarbonyl, C₂-C₆-Alkenyl oder C₂-C₆-Alkinyl substituiertes Pyrazolyl, Oxazolyl, Thiazolyl, Oxadiazolyl, Triazolyl, Pyridinyl, Pyridazinyl, Pyrimidinyl, Pyrazinyl oder Triazinyl steht.

3. Verbindungen der Formel (I) gemäß Anspruch 1, worin
A¹ für Wasserstoff, Halogen oder Cyano steht,
A² für Wasserstoff steht,
G¹ für N oder C-A¹ steht,
G² für einen Rest aus der Reihe 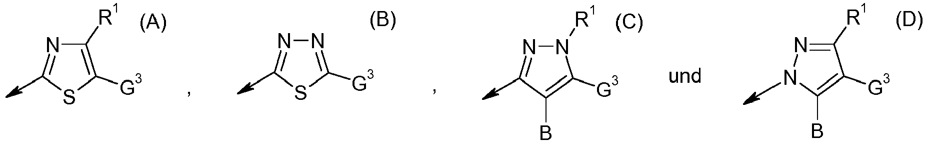 steht, worin der Pfeil die Bindung zum benachbarten Ring markiert,
R¹ für Wasserstoff oder C₁-C₄-Alkyl steht,
B für Wasserstoff steht,
G³ für jeweils gegebenenfalls durch Halogen, Cyano, Nitro, Amino, C₁-C₆-Alkylamino, Di-C₁-C₆-alkylamino, C₁-C₆-Alkyl, C₃-C₆-Cycloalkyl, C₁-C₆-Halogenalkyl, Hydroxy, C₁-C₆-Alkoxy, C₁-C₆-Halogenalkoxy, C₁-C₆-Alkylthio, C₁-C₆-Alkylcarbonyl, C₁-C₆-Alkoxycarbonyl, C₂-C₆-Alkenyl oder C₂-C₆-Alkinyl substituiertes 5-gliedriges Heteroaryl oder 6-gliedriges Heteroaryl steht,
G⁴ für den Rest 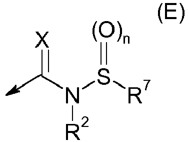 steht, worin der Pfeil die Bindung zu G³ markiert,
X für Sauerstoff steht,
n für 2 steht,
R² für einen Rest aus der Reihe Wasserstoff, C₁-C₄-Alkyl, C₂-C₄-Alkinyl, Cyano-C₁-C₄-alkyl und C₁-C₄-Alkoxy-C₁-C₄-alkyl, gegebenenfalls durch Halogen substituiertes C₁-C₄-Alkylcarbonyl oder C₁-C₄-Alkylsulfonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein ein- oder zweiwertiges Metallion oder ein gegebenenfalls durch C₁-C₄-Alkyl oder Aryl-C₁-C₄-alkyl substituiertes Ammonium-Ion steht,
R⁷ für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl und Heteroaryl-C₁-C₄-alkyl oder für NR' R" steht, worin R' und R" unabhängig voneinander jeweils für einen Rest aus der Reihe Wasserstoff, C₁-C₄-Alkyl und C₃-C₆-Cycloalkyl-C₁-C₄-alkyl stehen,
und worin Halogen ausgewählt ist aus der Reihe Fluor, Chlor, Brom und Iod, Aryl (auch als Teil einer größeren Einheit, wie beispielsweise Arylalkyl) ausgewählt ist aus der Reihe Phenyl, Naphthyl, Anthryl, Phenanthrenyl und Hetaryl (auch als Teil einer größeren Einheit, wie beispielsweise Hetarylalkyl) ausgewählt ist aus der Reihe Pyridyl, Pyrimidyl, Oxadiazolyl, Oxazolyl, Pyrazinyl, Imidazolyl, Thiazolyl, Thiadiazolyl und Furanyl.

4. Verbindungen der Formel (I) gemäß Anspruch 1, worin
A¹ für einen Rest aus der Reihe Wasserstoff, Fluor und Chlor steht,
A² für Wasserstoff steht,
G¹ für N oder C-A¹ steht, was zu Verbindungen führt, die die folgenden Strukturelemente enthalten: 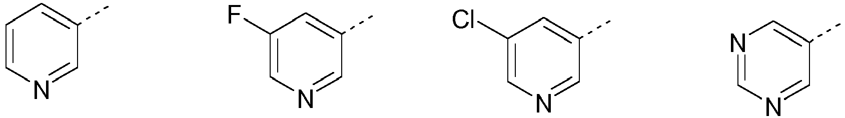 worin die gestrichelte Linie die Bindung zu G² bedeutet,
G² für einen Rest aus der Reihe 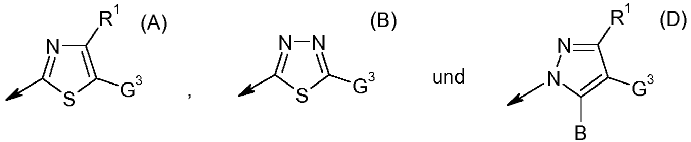 steht, worin der Pfeil jeweils die Bindung zum benachbarten Ring markiert,
R¹ für Wasserstoff oder Methyl steht,
B für Wasserstoff steht,
G³ für einen gegebenenfalls durch Halogen, Cyano, Methyl, Methoxy, Trifluoromethyl, Amino oder Dimethylamino substituierten Rest aus der Reihe 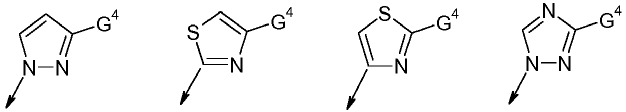 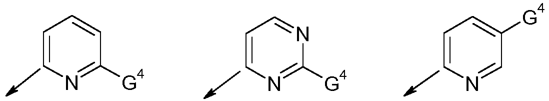 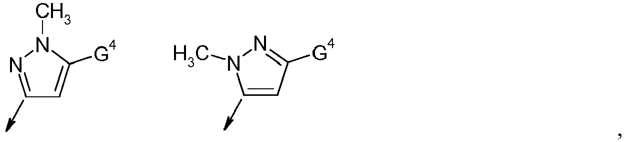 steht, worin der Pfeil jeweils die Bindung zu G² markiert und auch G⁴ zur Veranschaulichung eingezeichnet ist,
G⁴ für den Rest 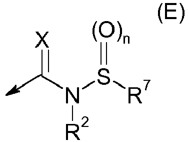 steht,
worin der Pfeil die Bindung zu G³ markiert,
X für Sauerstoff steht,
n für 2 steht,
R² für einen Rest aus der Reihe Wasserstoff, C₁-C₄-Alkyl, C₂-C₄-Alkinyl, Cyano-C₁-C₄-alkyl und C₁-C₄-Alkoxy-C₁-C₄-alkyl, gegebenenfalls durch Halogen substituiertes C₁-C₄-Alkylcarbonyl oder C₁-C₄-Alkylsulfonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein ein- oder zweiwertiges Metallion oder ein gegebenenfalls durch C₁-C₄-Alkyl oder Aryl-C₁-C₄-alkyl substituiertes Ammonium-Ion steht,.
R⁷ für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl und C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C1-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl und Heteroaryl-C₁-C₄-alkyl oder für NR' R" steht, worin R' und R" unabhängig voneinander jeweils für einen Rest aus der Reihe Wasserstoff und C₁-C₄-Alkyl stehen und worin Halogen ausgewählt ist aus der Reihe Fluor, Chlor und Brom, Aryl für Phenyl und
Hetaryl (gleichbedeutend mit Heteroaryl, auch als Teil einer größeren Einheit, wie beispielsweise Hetarylalkyl) für einen Rest ausgewählt aus der Reihe Pyrazolyl, Oxazolyl, Thiazolyl, Pyridyl, Pyrimidinyl, Pyridazinyl und Pyrazinyl steht.

5. Verbindungen der Formel (I) gemäß Anspruch 1, wobei die Verbindungen der Formel (I) Verbindungen der Formel (IA) 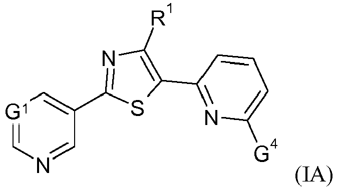 sind, worin
G¹ für C-H, C-F, C-C1 oder N steht,
R¹ für Wasserstoff oder Methyl steht und
G⁴ für den in Anspruch 1 genannten Rest (E) steht.

6. Verbindung der Formel (I) gemäß Anspruch 1, wobei die Verbindung der Formel (I) die Verbindung der Formel 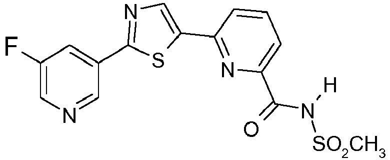 ist.

7. Verbindung der Formel gemäß Anspruch 1, wobei die Verbindung der Formel (I) die Verbindung der Formel 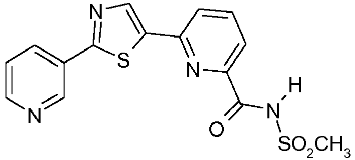 ist.

8. Verbindungen der Formel (I) gemäß Anspruch 1, wobei die Verbindungen der Formel (I) Verbindungen der Formel (IB) 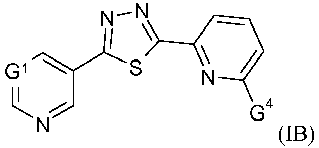 sind, worin
G¹ für C-H, C-F, C-Cl oder N steht und
G⁴ für den in Anspruch 1 genannten Rest (E) steht.

9. Verbindungen der Formel (I) gemäß Anspruch 1, wobei die Verbindungen der Formel (I) Verbindungen der Formel (IC) 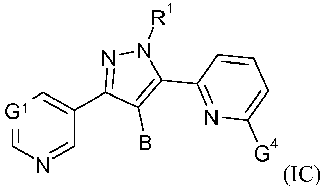 sind, worin
G¹ für C-H, C-F, C-Cl oder N steht,
R¹ für Wasserstoff oder Methyl steht,
B für Wasserstoff steht und
G⁴ für den in Anspruch 1 genannten Rest (E) steht.

10. Verbindungen der Formel (I) gemäß Anspruch 1, wobei die Verbindungen der Formel (I) Verbindungen der Formel (ID) 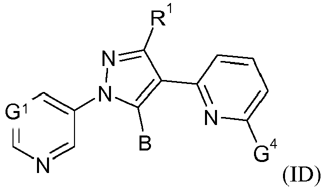 sind, worin
G¹ für C-H, C-F, C-Cl oder N steht,
R¹ für Wasserstoff oder Methyl steht,
B für Wasserstoff steht und
G⁴ für den in Anspruch 1 genannten Rest (E) steht.

11. Mittel, **gekennzeichnet durch** einen Gehalt von mindestens einer Verbindung der Formel (I) gemäß Ansprüchen 1 bis 9.

12. Verfahren zum Bekämpfen von Schädlingen **dadurch gekennzeichnet, dass** man eine Verbindung der Formel (I) gemäß Ansprüchen 1 bis 10 oder ein Mittel gemäß Anspruch 11 auf die Schädlinge und/oder ihren Lebensraum einwirken lässt, wobei Verfahren am tierischen oder menschlichen Körper ausgenommen sind.

13. Verbindungen der Formel (XII) 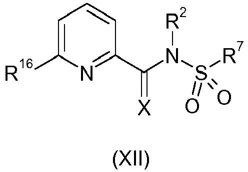 worin
R¹⁶ für Fluor, Chlor, Brom oder Iod steht,
X und R² die in Anspruch 1 angegebenen Bedeutungen haben und
R⁷ für einen Rest aus der Reihe jeweils gegebenenfalls durch Halogen, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl, C₃-C₆-Cycloalkyl-C₁-C₆-Alkyl und C₃-C₆-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heteroaryl und Heteroaryl-C₁-C₆-alkyl oder für NR'R" steht, worin R' und R" unabhängig voneinander jeweils für einen Rest aus der Reihe Wasserstoff, C₁-C₆-Alkyl, C₁-C₆-Haloalkyl, C₃-C₆-Cycloalkyl, C₃-C₆-Cycloalkyl-C₁-C₆-alkyl, C₁-C₆-Alkoxy, C₁-C₆-Alkylcarbonyl und C₁-C₆-Alkoxylcarbonyl stehen und worin Heteroaryl die in Anspruch 1 angegebenen Bedeutungen hat.

14. Verbindungen der Formel (XV) 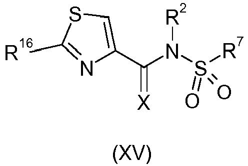 worin
R¹⁶ für Fluor, Chlor, Brom oder Iod steht und
X und R² und R⁷ die in Anspruch 1 angegebenen Bedeutungen haben und
R⁷ für einen Rest aus der Reihe jeweils gegebenenfalls durch Halogen, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl und C₁-C₆-Halogenalkylsulfonyl substituiertes C₁-C₆-Alkyl, C₂-C₆-Alkenyl und C₂-C₆-Alkinyl, jeweils gegebenenfalls durch Halogen, C₁-C₆-Alkyl, C₁-C₆-Halogenalkyl, C₁-C₆-Alkoxy oder C₁-C₆-Halogenalkoxy substituiertes C₃-C₆-Cycloalkyl, C₃-C₆-Cycloalkyl-C₁-C₆-Alkyl und C₃-C₆-Cycloalkenyl, in welchen die Ringe mindestens ein Heteroatom aus der Reihe Schwefel, Sauerstoff (wobei Sauerstoffatome nicht unmittelbar benachbart sein dürfen)
und Stickstoff enthalten können, jeweils gegebenenfalls durch Halogen, Cyano (auch im Alkylteil), Nitro, C₁-C₆-Alkyl, C₁-C₆-Halogenalkyl, C₃-C₆-Cycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heteroaryl, Aryl-C₁-C₆-alkyl und Heteroaryl-C₁-C₆-alkyl oder für NR'R" steht, worin R' und R" unabhängig voneinander jeweils für einen Rest aus der Reihe Wasserstoff, C₁-C₆-Alkyl, C₁-C₆-Haloalkyl, C₃-C₆-Cycloalkyl, C₃-C₆-Cycloalkyl-C₁-C₆-alkyl, C₁-C₆-Alkoxy, C₁-C₆-Alkylcarbonyl und C₁-C₆-Alkoxylcarbonyl stehen und worin Aryl und Heteroaryl die in Anspruch 1 angegebenen Bedeutungen haben.

15. Verbindungen der Formel (XVI) 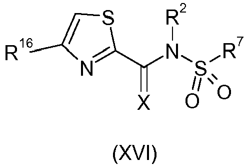 worin
R¹⁶ für Fluor, Chlor, Brom oder Iod steht und
X und R² und R⁷ die in Anspruch 1 angegebenen Bedeutungen haben.

## Claims

1. Compounds of the formula (I) 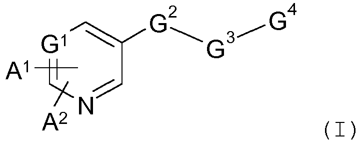 in which
A¹ and A² are each independently hydrogen, halogen, cyano, nitro, C₁-C₆-alkyl, C₃-C₆-cycloalkyl or C₁-C₆-alkoxy,
G¹ is N or C-A¹,
G² is a radical from the group of 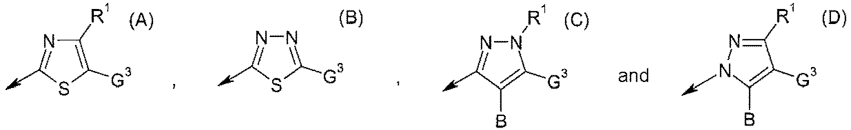 in which the arrow marks the bond to the adjacent ring,
R¹ in the case of the heterocycles (A) and (D) is hydrogen, halogen, cyano, C₁-C₆-alkyl, C₁-C₆-alkoxy or C₁-C₆-haloalkyl,
R¹ in the case of heterocycle (C) is hydrogen, C₁-C₆-alkyl or C₁-C₆-haloalkyl,
B is hydrogen, halogen, cyano, nitro, C₁-C₆-alkyl, C₁-C₆-haloalkyl, C₁-C₆-alkoxy or C₃-C₆-cycloalkyl,
G³ is in each case optionally halogen-, cyano-, nitro-, amino-, C₁-C₆-alkylamino-, di(C₁-C₆)-alkylamino-, C₁-C₆-alkyl-, C₃-C₆-cycloalkyl-, C₁-C₆-haloalkyl-, hydroxyl-, C₁-C₆-alkoxy-, C₁-C₆-haloalkoxy-, C₁-C₆-alkylthio-, C₁-C₆-alkylcarbonyl-, C₁-C₆-alkoxycarbonyl-, C₂-C₆-alkenyl- or C₂-C₆-alkynyl-substituted 5-membered heteroaryl or 6-membered heteroaryl,
G⁴ is a radical from the group of 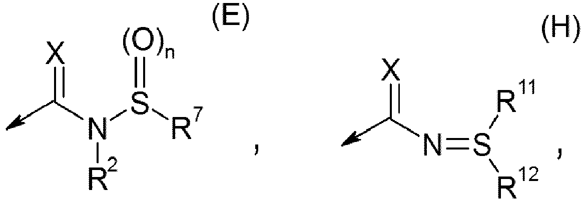
X is oxygen or sulphur,
n is 1 or 2,
R² is a radical from the group of hydrogen, C₁-C₆-alkyl, C₁-C₆-haloalkyl, cyano-C₁-C₆-alkyl, C₁-C₆-alkoxy, C₁-C₆-haloalkoxy, C₂-C₆-alkenyl, C₂-C₆-alkynyl, C₁-C₆-alkoxy-C₁-C₆-alkyl, in each case optionally halogen-substituted C₁-C₆-alkylcarbonyl and C₁-C₆-alkylsulphonyl, optionally halogen-substituted C₁-C₆-alkoxycarbonyl, optionally halogen-, C₁-C₆-alkyl-, C₁-C₆-alkoxy-, C₁-C₆-haloalkyl- and cyano-substituted C₃-C₆-cycloalkylcarbonyl, or a mono- or divalent metal ion or an optionally C₁-C₆-alkyl- or aryl-C₁-C₆-alkyl-substituted ammonium ion,
R⁷ is a radical from the group of in each case optionally halogen-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl- and C₁-C₆-haloalkylsulphonylsubstituted C₁-C₆-alkyl, C₂-C₆-alkenyl and C₂-C₆-alkynyl, in each case optionally halogen-, C₁-C₆-alkyl-, C₁-C₆-haloalkyl-, C₁-C₆-alkoxy- or C₁-C₆-haloalkoxy-substituted C₃-C₆-cycloalkyl, C₃-C₆-cycloalkyl-C₁-C₆-alkyl and C₃-C₆-cycloalkenyl, in which the rings may contain at least one heteroatom from the group of sulphur, oxygen (where oxygen atoms must not be immediately adjacent) and nitrogen, in each case optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl-, C₁-C₆-haloalkylsulphonyl-, amino-, C₁-C₆-alkylamino-, di(C₁-C₆-alkyl)amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted aryl, heteroaryl, aryl-C₁-C₆-alkyl and heteroaryl-C₁-C₆-alkyl, or NR'R" in which R' and R" are each independently a radical from the group of hydrogen, C₁-C₆-alkyl, C₁-C₆-haloalkyl, C₃-C₆-cycloalkyl, C₃-C₆-cycloalkyl-C₁-C₆-alkyl, C₁-C₆-alkoxy, C₁-C₆-alkylcarbonyl and C₁-C₆-alkoxylcarbonyl,
R² and R⁷ may also, together with the N-S(O)ₙ group to which they are bonded, form a saturated or unsaturated and optionally halogen-, C₁-C₆-alkyl-, C₁-C₆-haloalkyl-, C₁-C₆-alkoxy-, C₁-C₆-haloalkoxy-substituted 5- to 7-membered ring which may contain one or more further heteroatoms from the group of sulphur, oxygen (where oxygen atoms must not be immediately adjacent) and nitrogen and/or at least one carbonyl group,
R¹¹ and R¹² are each independently an in each case optionally halogen-, C₁-C₆-alkyl-, C₁-C₆-haloalkyl-, C₁-C₆-alkoxy-, C₁-C₆-haloalkoxy-substituted radical from the group of C₁-C₆-alkyl, C₂-C₆-alkenyl, C₂-C₆-alkynyl, phenyl and phenyl-C₁-C₆-alkyl,
and wherein
halogen is selected from the group of fluorine, chlorine, bromine and iodine, aryl (including as part of a larger unit, for example arylalkyl) is selected from the group of phenyl, naphthyl, anthryl, phenanthrenyl, hetaryl (synonymous with heteroaryl, including as part of a larger unit, for example hetarylalkyl) is selected from the group of furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolinyl, cinnolinyl, phthalazinyl, quinazolinyl, quinoxalinyl, naphthyridinyl, benzotriazinyl, purinyl, pteridinyl and indolizinyl,
and salts and N-oxides of the compounds of the formula (I).

2. Compounds of the formula (I) according to Claim 1, in which
G³ is in each case optionally halogen-, cyano-, nitro-, amino-, C₁-C₆₋alkylamino-, di-(C₁-C₆)-alkylamino-, C₁-C₆-alkyl-, C₃-C₆-cycloalkyl-, C₁-C₆-haloalkyl-, hydroxyl-, C₁-C₆-alkoxy-, C₁-C₆-haloalkoxy-, C₁-C₆-alkylthio-, C₁-C₆-alkylcarbonyl-, C₁-C₆-alkoxycarbonyl-, C₂-C₆-alkenyl- or C₂-C₆-alkynyl-substituted pyrazolyl, oxazolyl, thiazolyl, oxadiazolyl, triazolyl, pyridinyl, pyridazinyl, pyrimidinyl, pyrazinyl or triazinyl.

3. Compounds of the formula (I) according to Claim 1, in which
A¹ is hydrogen, halogen or cyano,
A² is hydrogen,
G¹ is N or C-A¹,
G² is a radical from the group of 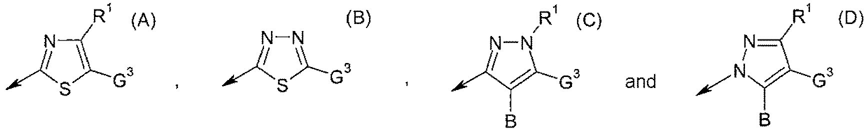 in which the arrow marks the bond to the adjacent ring,
R¹ is hydrogen or C₁-C₄-alkyl,
B is hydrogen,
G³ is in each case optionally halogen-, cyano-, nitro-, amino-, C₁-C₆-alkylamino-, di-C₁-C₆-alkylamino-, C₁-C₆-alkyl-, C₃-C₆-cycloalkyl-, C₁-C₆-haloalkyl-, hydroxyl-, C₁-C₆-alkoxy-, C₁-C₆-haloalkoxy-, C₁-C₆-alkylthio-, C₁-C₆-alkylcarbonyl-, C₁-C₆-alkoxycarbonyl-, C₂-C₆-alkenyl- or C₂-C₆-alkynyl-substituted 5-membered heteroaryl or 6-membered heteroaryl,
G⁴ is the radical 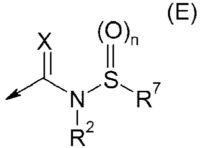 in which the arrow marks the bond to G³,
X is oxygen,
n is 2,
R² is a radical from the group of hydrogen, C₁-C₄-alkyl, C₂-C₄-alkynyl, cyano-C₁-C₄alkyl and C₁-C₄-alkoxy-C₁-C₄-alkyl, optionally halogen-substituted C₁-C₄-alkylcarbonyl or C₁-C₄-alkylsulphonyl, optionally halogen-substituted C₁-C₄-alkoxycarbonyl, optionally halogen-, C₁-C₄-alkyl-, C₁-C₄-alkoxy-, C₁-C₄-haloalkyl- and cyano-substituted C₃-C₆-cycloalkylcarbonyl, or a mono- or divalent metal ion or an optionally C₁-C₄-alkyl- or aryl-C₁-C₄-alkyl-substituted ammonium ion,
R⁷ is a radical from the group of in each case optionally halogen-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl- and C₁-C₄-haloalkylsulphonyl-substituted C₁-C₄-alkyl, C₂-C₄-alkenyl and C₂-C₄-alkynyl, in each case optionally halogen-, C₁-C₄-alkyl-, C₁-C₄-haloalkyl-, C₁-C₄-alkoxy-, C₁-C₄-haloalkoxy-substituted C₃-C₆-cycloalkyl, C₃-C₆-cycloalkyl-C₁-C₄-alkyl and C₃-C₄-cycloalkenyl, in which the rings may contain at least one heteroatom from the group of sulphur, oxygen (where oxygen atoms must not be immediately adjacent) and nitrogen, in each case optionally halogen-, cyano- (including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl-, C₁-C₄-haloalkylsulphonyl-, amino-, C₁-C₄-alkylamino-, di(C₁-C₄-alkyl)amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted aryl, heteroaryl, aryl-C₁-C₄-alkyl and heteroaryl-C₁-C₄-alkyl or NR'R'' in which R' and R'' are each independently a radical from the group of hydrogen, C₁-C₄-alkyl and C₃-C₆-cycloalkyl-C₁-C₄-alkyl,
and in which halogen is selected from the group of fluorine, chlorine, bromine and iodine, aryl (including as part of a larger unit, for example arylalkyl) is selected from the group of phenyl, naphthyl, anthryl, phenanthrenyl, and hetaryl (including as part of a larger unit, for example hetarylalkyl) is selected from the group of pyridyl, pyrimidyl, oxadiazolyl, oxazolyl, pyrazinyl, imidazolyl, thiszolyl, thiadiazolyl and furanyl.

4. Compounds of the formua (I) according to Claim 1, in which
A¹ is a radical from the group of hydrogen, fluorine and chlorine,
A² is hydrogen,
G¹ is N or C-A¹, which leads to compounds containing the following structural elements: 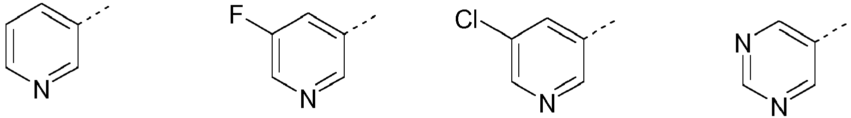 in which the broken line means the bond to G²,
G² is a radical from the group of 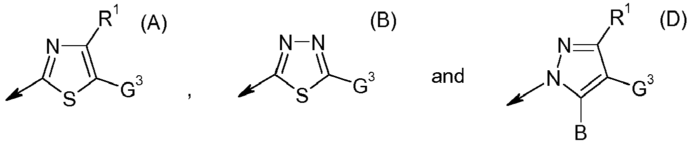 in which the arrow in each case marks the bond to the adjacent ring,
R¹ is hydrogen or methyl,
B is hydrogen,
G³ is an optionally halogen-, cyano-, methyl-, methoxy-, trifluoromethyl-, amino- or dimethylamino-substituted radical from the group of 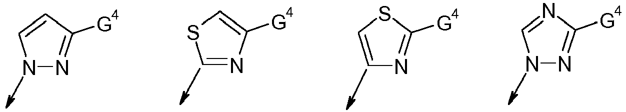 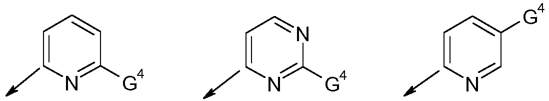 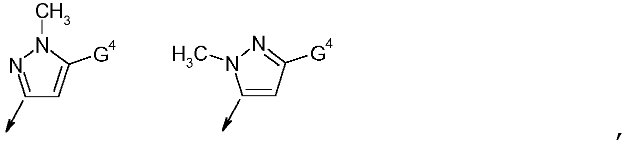 in which the arrow in each case marks the bond to G², and G⁴ is also shown for illustration,
G⁴ is the radical 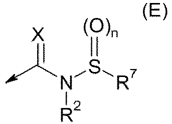 in which the arrow marks the bond to G³,
X is oxygen,
n is 2,
R² is a radical from the group of hydrogen, C₁-C₄-alkyl, C₂-C₄-alkynyl, cyano-C₁-C₄-alkyl and C₁-C₄-alkoxy-C₁-C₄-alkyl, optionally halogen-substituted C₁-C₄-alkylcarbonyl or C₁-C₄-alkylsulfonyl, optionally halogen-substituted C₁-C₄-alkoxycarbonyl, optionally halogen-, C₁-C₄-alkyl-, C₁-C₄-alkoxy-, C₁-C₄-halogenalkyl- and cyano-substituted C₃-C₆-cycloalkylcarbonyl, or a mono- or divalent metal ion or an optionally C₁-C₄-alkyl- or aryl-C₁-C₄-alkyl-substituted ammonium ion,
R⁷ is a radical from the group of in each case optionally halogen-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl- and C₁-C₄-haloalkylsulphonyl-substituted C₁-C₄-alkyl, C₂-C₄-alkenyl and C₂-C₄-alkynyl, in each case optionally halogen-, C₁-C₄-alkyl, C₁-C₄-haloalkyl-, C₁-C₄-alkoxy-, C₁-C₄-haloalkoxy-substituted C₃-C₆-cycloalkyl, C₃-C₆-cycloalkyl-C₁-C₄-alkyl and C₃-C₄-cycloalkenyl, in which the rings may contain at least one heteroatom from the group of sulphur, oxygen (where oxygen atoms must not be immediately adjacent) and nitrogen, in each case optionally halogen-, cyano-(including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl-, C₁-C₄-haloalkylsulphonyl-, amino-, C₁-C₄-alkylamino-, di(C₁-C₄-alkyl)amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted aryl, heteroaryl, aryl-C₁-C₄-alkyl and heteroaryl-C₁-C₄-alkyl, or NR'R'' in which R' and R'' are each independently a radical from the group of hydrogen and C₁-C₄-alkyl and in which halogen is selected from the group of fluorine, chlorine and bromine, aryl is phenyl and hetaryl (equivalent to heteroaryl, including as part of a larger unit, for example hetarylalkyl) is a radical selected from the group of pyrazolyl, oxazolyl, thiazolyl, pyridyl, pyrimidinyl, pryidazinyl and pyrazinyl.

5. Compounds of the formula (I) according to Claim 1, wherein the compounds of the formula (I) are compounds of the formula (IA) 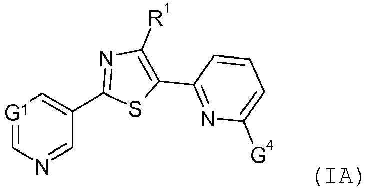 in which
G¹ is C-H, C-F, C-Cl or N,
R¹ is hydrogen or methyl and
G⁴ is the (E) radical specified in Claim 1.

6. Compound of the formula (I) according to Claim 1, wherein the compound of the formula (I) is the compound of the formula

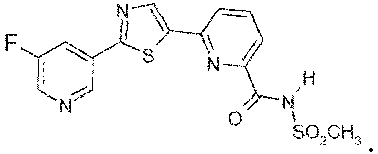

7. Compound of the formula according to Claim 1, wherein the compound of the formula (I) is the compound of the formula

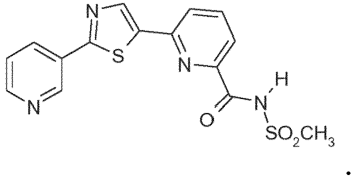

8. Compounds of the formula (I) according to Claim 1, wherein the compounds of the formula (I) are compounds of the formula (IB) 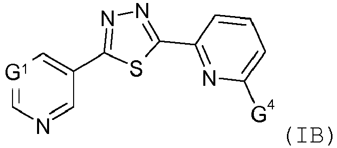 in which
G¹ is C-H, C-F, C-Cl or N and
G⁴ is the (E) radical specified in Claim 1.

9. Compounds of the formula (I) according to Claim 1, wherein the compounds of the formula (I) are compounds of the formula (IC) 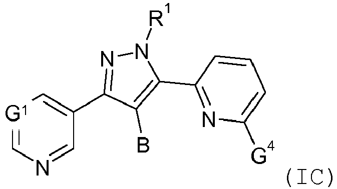 in which
G¹ is C-H, C-F, C-Cl or N,
R¹ is hydrogen or methyl,
B is hydrogen and
G⁴ is the (E) radical specified in Claim 1.

10. Compounds of the formula (I) according to Claim 1, wherein the compounds of the formula (I) are compounds of the formula (ID) 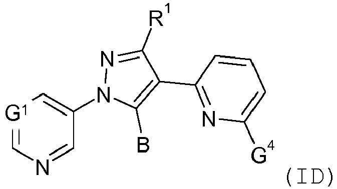 in which
G¹ is C-H, C-F, C-Cl or N,
R¹ is hydrogen or methyl,
B is hydrogen and
G⁴ is the (E) radical specified in Claim 1.

11. Composition **characterized by** a content of at least one compound of the formula (I) according to Claims 1 to 9.

12. Method for controlling pests, **characterized in that** a compound of the formula (I) according to Claims 1 to 10 or a composition according to Claim 11 is allowed to act on the pests and/or their habitat, excluding methods on the animal or human body.

13. Compounds of the formula (XII) 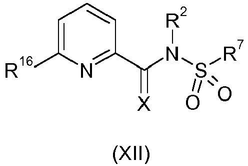 in which
R¹⁶ is fluorine, chlorine, bromine or iodine,
X and R² are each as defined in Claim 1 and
R⁷ is a radical from the group of in each case optionally halogen-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl- and C₁-C₆-haloalkylsulphonyl-substituted C₁-C₆-alkyl, C₂-C₆-alkenyl and C₂-C₆-alkynyl, in each case optionally halogen-, C₁-C₆-alkyl-, C₁-C₆-haloalkyl-,. C₁-C₆-alkoxy- or C₁-C₆-haloalkoxy-substituted C₃-C₆-cycloalkyl, C₃-C₆-cycloalkyl-C₁-C₆-alkyl and C₃-C₆-cycloalkenyl, in which the rings may contain at least one heteroatom from the group of sulphur, oxygen (where oxygen atoms must not be immediately adjacent) and nitrogen, in each case optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl-, C₁-C₆-haloalkylsulphonyl-, amino-, C₁-C₆-alkylamino-, di(C₁-C₆-alkyl)amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl substituted heteroaryl and heteroaryl-C₁-C₆-alkyl, or NR'R" in which R' and R" are each independently a radical from the group of hydrogen, C₁-C₆-alkyl, C₁-C₆-haloalkyl, C₃-C₆-cycloalkyl, C₃-C₆-cycloalkyl-C₁-C₆-alkyl, C₁-C₆-alkoxy, C₁-C₆-alkylcarbonyl and C₁-C₆-alkoxylcarbonyl and in which heteroaryl is as defined in Claim 1.

14. Compounds of the formula (XV) 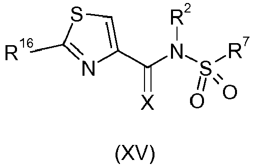 in which
R¹⁶ is fluorine, chlorine, bromine or iodine and
X and R² and R⁷ are each as defined in Claim 1 and
R⁷ is a radical from the group of in each case optionally halogen-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl- and C₁-C₆-haloalkylsulphonyl-substituted C₁-C₆-alkyl, C₂-C₆-alkenyl and C₂-C₆-alkynyl, in each case optionally halogen-, C₁-C₆-alkyl-, C₁-C₆-haloalkyl-, C₁-C₆-alkoxy- or C₁-C₆-haloalkoxy-substituted C₃-C₆-cycloalkyl, C₃-C₆-cycloalkyl-C₁-C₆-alkyl and C₃-C₆-cycloalkenyl, in which the rings may contain at least one heteroatom from the group of sulphur, oxygen (where oxygen atoms must not be immediately adjacent) and nitrogen, in each case optionally halogen-, cyano- (including in the alkyl moiety), nitro-, C₁-C₆-alkyl-, C₁-C₆-haloalkyl-, C₃-C₆-cycloalkyl-, C₁-C₆-alkoxy-, C₁-C₆-haloalkoxy-, C₁-C₆-alkylthio-, C₁-C₆-haloalkylthio-, C₁-C₆-alkylsulphinyl-, C₁-C₆-haloalkylsulphinyl-, C₁-C₆-alkylsulphonyl-, C₁-C₆-haloalkylsulphonyl-, amino-, C₁-C₆-alkylamino-, di(C₁-C₆-alkyl)amino-, C₁-C₆-alkylcarbonylamino-, C₁-C₆-alkoxycarbonylamino-, C₁-C₆-alkoxy-C₁-C₆-alkyl-, C₁-C₆-haloalkoxy-C₁-C₆-alkyl-, C₂-C₆-alkenyl-, C₂-C₆-alkynyl-, C₃-C₆-cycloalkyl-C₁-C₆-alkyl-, C₁-C₆-alkylcarbonyl-, C₁-C₆-alkoxycarbonyl- or aminocarbonyl-substituted heteroaryl, aryl-C₁-C₆-alkyl and heteroaryl-C₁-C₆-alkyl, or NR'R'' in which R' and R" are each independently a radical from the group of hydrogen, C₁-C₆-alkyl, C₁-C₆-haloalkyl, C₃-C₆-cycloalkyl, C₃-C₆-cycloalkyl-C₁-C₆-alkyl, C₁-C₆-alkoxy, C₁-C₆-alkylcarbonyl and C₁-C₆-alkoxylcarbonyl and in which aryl and heteroaryl are each as defined in Claim 1.

15. Compounds of the formula (XVI) 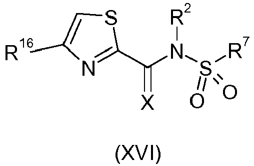 in which R¹⁶ is fluorine, chlorine, bromine or iodine and X and R² and R⁷ are each as defined in Claim 1.

## Revendications

1. Composés de formule (I) 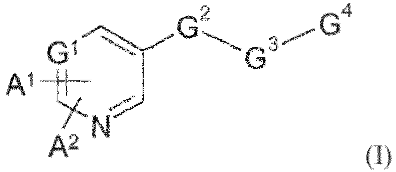 dans laquelle
A¹ et A² représentent indépendamment l'un de l'autre hydrogène, halogène, cyano, nitro, alkyle en C₁-C₆, cycloalkyle en C₃-C₆ ou alcoxy en C₁-C₆,
G¹ représente N ou C-A¹,
G² représente un radical de la série 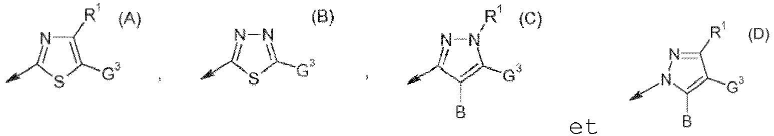 dans lesquels la flèche indique la liaison au cycle voisin,
R¹ dans le cas des hétérocycles (A) et (D), représente hydrogène, halogène, cyano, alkyle en C₁-C₆, alcoxy en C₁-C₆ ou haloalkyle en C₁-C₆,
R¹ dans le cas de l'hétérocycle (C), représente hydrogène, alkyle en C₁-C₆ ou haloalkyle en C₁-C₆,
B représente hydrogène, halogène, cyano, nitro, alkyle en C₁-C₆, haloalkyle en C₁-C₆, alcoxy en C₁-C₆ ou cycloalkyle en C₃-C₆,
G³ représente hétéroaryle à 5 éléments ou hétéroaryle à 6 éléments, chacun éventuellement substitué par halogène, cyano, nitro, amino, alkylamino en C₁-C₆, dialkylamino en (C₁-C₆), alkyle en C₁-C₆, cycloalkyle en C₃-C₆, halogénoalkyle en C₁-C₆, hydroxy, alcoxy en C₁-C₆, halogénoalcoxy en C₁-C₆, alkylthio en C₁-C₆, alkylcarbonyle en C₁-C₆, alcoxycarbonyle en C₁-C₆, alcényle en C₂-C₆ ou alcynyle en C₂-C₆,
G⁴ représente un radical de la série 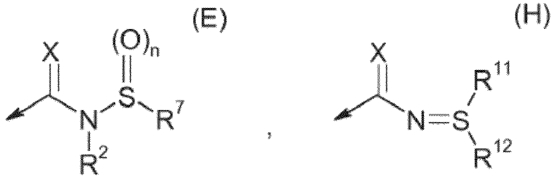
X représente oxygène ou soufre,
n représente 1 ou 2,
R² représente un radical de la série constituée par hydrogène, alkyle en C₁-C₆, halogénoalkyle en C₁-C₆, cyano-alkyle en C₁-C₆, alcoxy en C₁-C₆, halogénoalcoxy en C₁-C₆, alcényle en C₂-C₆, alcynyle en C₂-C₆, alcoxy en C₁-C₆-alkyle en C₁-C₆ ; alkylcarbonyle en C₁-C₆ et alkylsulfonyle en C₁-C₆, chacun éventuellement substitué par halogène ; alcoxycarbonyle en C₁-C₆ éventuellement substitué par halogène ; cycloalkylcarbonyle en C₃-C₆ éventuellement substitué par halogène, alkyle en C₁-C₆, alcoxy en C₁-C₆, halogénoalkyle en C₁-C₆ et cyano, ou un ion métallique mono- ou bivalent, ou un ion ammonium éventuellement substitué par alkyle en C₁-C₆ ou aryl-alkyle en C₁-C₆,
R⁷ représente un radical de la série constituée par alkyle en C₁-C₆, alcényle en C₂-C₆ et alcynyle en C₂-C₆, chacun éventuellement substitué par halogène, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆ et halogénoalkylsulfonyle en C₁-C₆; cycloalkyle en C₃-C₆, cycloalkyle en C₃-C₆-alkyle en C₁-C₆ et cycloalcényle en C₃-C₆, chacun éventuellement substitué par halogène, alkyle en C₁-C₆, halogénoalkyle en C₁-C₆, alcoxy en C₁-C₆ ou halogénoalcoxy en C₁-C₆, dans lesquels les cycles peuvent contenir au moins un hétéroatome de la série constituée par soufre, oxygène (des atomes d'oxygène ne pouvant pas être directement voisins) et azote ; aryle, hétéroaryle, aryl-alkyle en C₁-C₆ et hétéroaryl-alkyle en C₁-C₆, chacun éventuellement substitué par halogène, cyano (également dans la partie alkyle), nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, amino, alkylamino en C₁-C₆, di(alkyle en C₁-C₆)amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle ; ou NR'R", R' et R" représentant chacun indépendamment l'un de l'autre un radical de la série constituée par hydrogène, alkyle en C₁-C₆, haloalkyle en C₁-C₆, cycloalkyle en C₃-C₆, cycloalkyle en C₃-C₆-alkyle en C₁-C₆, alcoxy en C₁-C₆, alkylcarbonyle en C₁-C₆ et alcoxycarbonyle en C₁-C₆,
R² et R⁷ peuvent également former ensemble avec le groupe N-S(O)ₙ auquel ils sont reliés un cycle de 5 à 7 éléments saturé ou insaturé et éventuellement substitué par halogène, alkyle en C₁-C₆, halogénoalkyle en C₁-C₆, alcoxy en C₁-C₆, halogénoalcoxy en C₁-C₆, qui peut contenir un ou plusieurs hétéroatomes supplémentaires de la série constituée par soufre, oxygène (deux atomes d'oxygène ne pouvant pas être directement voisins) et azote et/ou au moins un groupe carbonyle,
R¹¹ et R¹² représentent indépendamment l'un de l'autre un radical de la série constituée par alkyle en C₁-C₆, alcényle en C₂-C₆, alcynyle en C₂-C₆, phényle et phényl-alkyle en C₁-C₆, chacun éventuellement substitué par halogène, alkyle en C₁-C₆, halogénoalkyle en C₁-C₆, alcoxy en C₁-C₆, halogénoalcoxy en C₁-C₆,
halogène étant choisi dans la série constituée par fluor, chlore, brome et iode ; aryle (également en tant que partie d'une unité plus grande, telle que par exemple arylalkyle) étant choisi dans la série constituée par phényle, naphtyle, anthryle, phénanthryle ; hétaryle (synonyme d'hétéroaryle, également en tant que partie d'une unité plus grande, telle que par exemple hétarylalkyle) étant choisi dans la série constituée par furyle, thiényle, pyrrolyle, pyrazolyle, imidazolyle, 1,2,3-triazolyle, 1,2,4-triazolyle, oxazolyle, isoxazolyle, thiazolyle, isothiazolyle, 1,2,3-oxadiazolyle, 1,2,4-oxadiazolyle, 1,3,4-oxadiazolyle, 1,2,5-oxadiazolyle, 1,2,3-thiadiazolyle, 1,2,4-thiadiazolyle, 1,3,4-thiadiazolyle, 1,2,5-thiadiazolyle, pyridyle, pyrimidinyle, pyridazinyle, pyrazinyle, 1,2,3-triazinyle, 1,2,4-triazinyle, 1,3,5-triazinyle, benzofuryle, benzoisofuryle, benzothiényle, benzisothiényle, indolyle, isoindolyle, indazolyle, benzothiazolyle, benzisothiazolyle, benzoxazolyle, benzisoxazolyle, benzimidazolyle, 2,1,3-benzoxadiazole, quinolinyle, isoquinolinyle, cinnolinyle, phthalazinyle, quinazolinyle, quinoxalinyle, naphtyridinyle, benzotriazinyle, purinyle, ptéridinyle et indolizinyle,
ainsi que les sels et les N-oxydes des composés de formule (I).

2. Composés de formule (I) selon la revendication 1, dans lesquels
G³ représente pyrazolyle, oxazolyle, thiazolyle, oxadiazolyle, triazolyle, pyridinyle, pyridazinyle, pyrimidinyle, pyrazinyle ou triazinyle, chacun éventuellement substitué par halogène, cyano, nitro, amino, alkylamino en C₁-C₆, di-alkylamino en (C₁-C₆), alkyle en C₁-C₆, cycloalkyle en C₃-C₆, halogénoalkyle en C₁-C₆, hydroxy, alcoxy en C₁-C₆, halogénoalcoxy en C₁-C₆, alkylthio en C₁-C₆, alkylcarbonyle en C₁-C₆, alcoxycarbonyle en C₁-C₆, alcényle en C₂-C₆ ou alcynyle en C₂-C₆.

3. Composés de formule (I) selon la revendication 1, dans lesquels
A¹ représente hydrogène, halogène ou cyano,
A² représente hydrogène,
G¹ représente N ou C-A¹,
G² représente un radical de la série 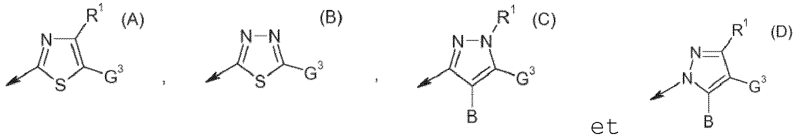
dans lesquels la flèche indique la liaison au cycle voisin,
R¹ représente hydrogène ou alkyle en C₁-C₄,
B représente hydrogène,
G³ représente hétéroaryle à 5 éléments ou hétéroaryle à 6 éléments, chacun éventuellement substitué par halogène, cyano, nitro, amino, alkylamino en C₁-C₆, dialkylamino en C₁-C₆, alkyle en C₁-C₆, cycloalkyle en C₃-C₆, halogénoalkyle en C₁-C₆, hydroxy, alcoxy en C₁-C₆, halogénoalcoxy en C₁-C₆, alkylthio en C₁-C₆, alkylcarbonyle en C₁-C₆, alcoxycarbonyle en C₁-C₆, alcényle en C₂-C₆ ou alcynyle en C₂-C₆,
G⁴ représente le radical 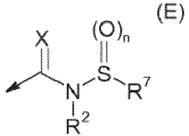
la flèche indiquant la liaison à G³,
X représente oxygène,
n représente 2,
R² représente un radical de la série constituée par hydrogène, alkyle en C₁-C₄, alcynyle en C₂-C₄, cyano-alkyle en C₁-C₄ et alcoxy en C₁-C₄-alkyle en C₁-C₄ ; alkylcarbonyle en C₁-C₄ et alkylsulfonyle en C₁-C₄, chacun éventuellement substitué par halogène ; alcoxycarbonyle en C₁-C₄ éventuellement substitué par halogène ; cycloalkylcarbonyle en C₃-C₆ éventuellement substitué par halogène, alkyle en C₁-C₄, alcoxy en C₁-C₄, halogénoalkyle en C₁-C₄ et cyano, ou un ion métallique mono- ou bivalent, ou un ion ammonium éventuellement substitué par alkyle en C₁-C₄ ou aryl-alkyle en C₁-C₄,
R⁷ représente un radical de la série constituée par alkyle en C₁-C₄, alcényle en C₂-C₄ et alcynyle en C₂-C₄, chacun éventuellement substitué par halogène, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄ et halogénoalkylsulfonyle en C₁-C₄ ; cycloalkyle en C₃-C₆, cycloalkyle en C₃-C₆-alkyle en C₁-C₄ et cycloalcényle en C₃-C₄, chacun éventuellement substitué par halogène, alkyle en C₁-C₄, halogénoalkyle en C₁-C₄, alcoxy en C₁-C₄ ou halogénoalcoxy en C₁-C₄, dans lesquels les cycles peuvent contenir au moins un hétéroatome de la série constituée par soufre, oxygène (des atomes d'oxygène ne pouvant pas être directement voisins) et azote ; aryle, hétéroaryle, aryl-alkyle en C₁-C₄ et hétéroaryl-alkyle en C₁-C₄, chacun éventuellement substitué par halogène, cyano (également dans la partie alkyle), nitro, alkyle en C₁-C₄, halogénoalkyle en C₁-C₄, cycloalkyle en C₃-C₆, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, amino, alkylamino en C₁-C₄, di(alkyle en C₁-C₄)amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle ; ou NR'R",
R' et R" représentant chacun indépendamment l'un de l'autre un radical de la série constituée par hydrogène, alkyle en C₁-C₄ et cycloalkyle en C₃-C₆-alkyle en C₁-C₄,
halogène étant choisi dans la série constituée par fluor, chlore, brome et iode ; aryle (également en tant que partie d'une unité plus grande, telle que par exemple arylalkyle) étant choisi dans la série constituée par phényle, naphtyle, anthryle, phénanthryle ; et hétaryle (également en tant que partie d'une unité plus grande, telle que par exemple hétarylalkyle) étant choisi dans la série constituée par pyridyle, pyrimidyle, oxadiazolyle, oxazolyle, pyrazinyle, imidazolyle, thiazolyle, thiadiazolyle et furanyle.

4. Composés de formule (I) selon la revendication 1, dans lesquels
A¹ représente un radical de la série constituée par hydrogène, fluor et chlore,
A² représente hydrogène,
G¹ représente N ou C-A¹, ce qui conduit à des composés qui contiennent les éléments structuraux suivantes : 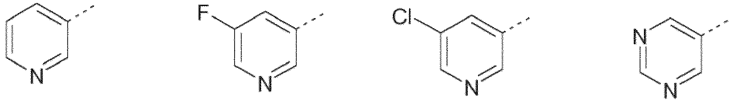 dans lesquels la ligne en pointillés signifie la liaison à G²,
G² représente un radical de la série 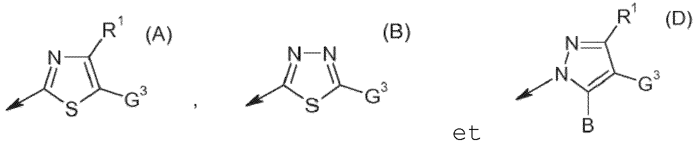 dans lesquels la flèche indique la liaison au cycle voisin,
R¹ représente hydrogène ou méthyle,
B représente hydrogène,
G³ représente un radical de la série 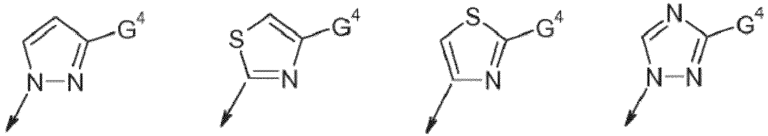 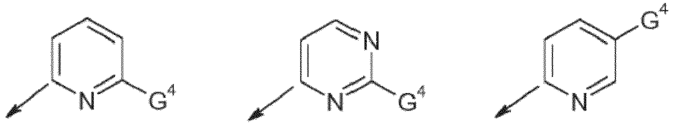 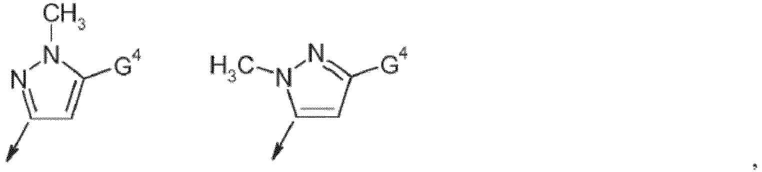 éventuellement substitué par halogène, cyano, méthyle, méthoxy, trifluorométhyle, amino ou diméthylamino, la flèche indiquant à chaque fois la liaison à G² et G⁴ étant également indiqué à titre d'illustration,
G⁴ représente le radical 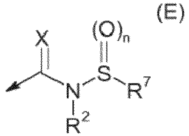
la flèche indiquant la liaison à G³,
X représente oxygène,
n représente 2,
R² représente un radical de la série constituée par hydrogène, alkyle en C₁-C₄, alcynyle en C₂-C₄, cyano-alkyle en C₁-C₄ et alcoxy en C₁-C₄-alkyle en C₁-C₄ ; alkylcarbonyle en C₁-C₄ ou alkylsulfonyle en C₁-C₄, éventuellement substitué par halogène ; alcoxycarbonyle en C₁-C₄ éventuellement substitué par halogène ; cycloalkylcarbonyle en C₃-C₆ éventuellement substitué par halogène, alkyle en C₁-C₄, alcoxy en C₁-C₄, halogénoalkyle en C₁-C₄ et cyano, ou un ion métallique mono- ou bivalent, ou un ion ammonium éventuellement substitué par alkyle en C₁-C₄ ou aryl-alkyle en C₁-C₄,
R⁷ représente un radical de la série constituée par alkyle en C₁-C₄, alcényle en C₂-C₄ et alcynyle en C₂-C₄, chacun éventuellement substitué par halogène, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄ et halogénoalkylsulfonyle en C₁-C₄ ; cycloalkyle en C₃-C₆, cycloalkyle en C₃-C₆-alkyle en C₁-C₄ et cycloalcényle en C₃-C₄, chacun éventuellement substitué par halogène, alkyle en C₁-C₄, halogénoalkyle en C₁-C₄, alcoxy en C₁-C₄ ou halogénoalcoxy en C₁-C₄, dans lesquels les cycles peuvent contenir au moins un hétéroatome de la série constituée par soufre, oxygène (des atomes d'oxygène ne pouvant pas être directement voisins) et azote ; aryle, hétéroaryle, aryl-alkyle en C₁-C₄ et hétéroaryl-alkyle en C₁-C₄, chacun éventuellement substitué par halogène, cyano (également dans la partie alkyle), nitro, alkyle en C₁-C₄, halogénoalkyle en C₁-C₄, cycloalkyle en C₃-C₆, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, amino, alkylamino en C₁-C₄, di(alkyle en C₁-C₄)amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle ; ou NR'R",
R' et R" représentant chacun indépendamment l'un de l'autre un radical de la série constituée par hydrogène et alkyle en C₁-C₄, et halogène étant choisi dans la série constituée par fluor, chlore et brome ; aryle représentant phényle ; et hétaryle (synonyme d'hétéroaryle, également en tant que partie d'une unité plus grande, telle que par exemple hétarylalkyle) représentant un radical choisi dans la série constituée par pyrazolyle, oxazolyle, thiazolyle, pyridyle, pyrimidinyle, pyridazinyle et pyrazinyle.

5. Composés de formule (I) selon la revendication 1, dans lesquels les composés de formule (I) sont des composés de formule (IA) 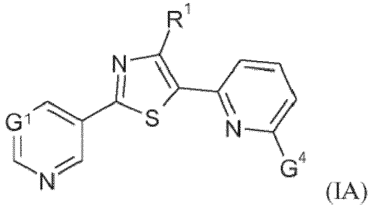 dans lesquels
G¹ représente C-H, C-F, C-Cl ou N,
R¹ représente hydrogène ou méthyle, et
G⁴ représente le radical (E) indiqué dans la revendication 1.

6. Composé de formule (I) selon la revendication 1, dans lequel le composé de formule (I) est le composé de formule

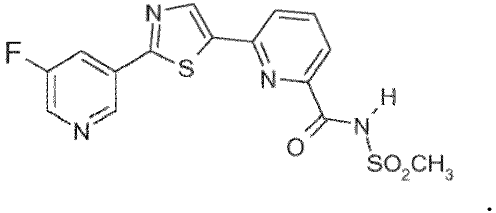

7. Composé de formule (I) selon la revendication 1, dans lequel le composé de formule (I) est le composé de formule

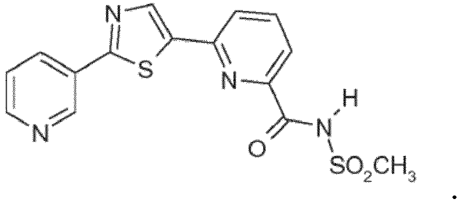

8. Composés de formule (I) selon la revendication 1, dans lesquels les composés de formule (I) sont des composés de formule (IB) 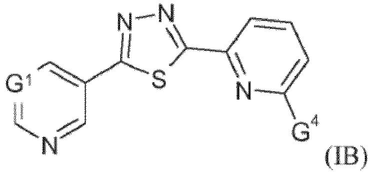 dans lesquels
G¹ représente C-H, C-F, C-Cl ou N, et
G⁴ représente le radical (E) indiqué dans la revendication 1.

9. Composés de formule (I) selon la revendication 1, dans lesquels les composés de formule (I) sont des composés de formule (IC) 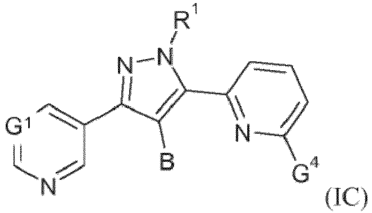 dans lesquels
G¹ représente C-H, C-F, C-Cl ou N,
R¹ représente hydrogène ou méthyle,
B représente hydrogène, et
G⁴ représente le radical (E) indiqué dans la revendication 1.

10. Composés de formule (I) selon la revendication 1, dans lesquels les composés de formule (I) sont des composés de formule (ID) 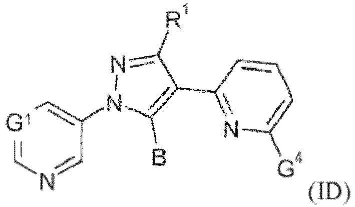 dans lesquels
G¹ représente C-H, C-F, C-Cl ou N,
R¹ représente hydrogène ou méthyle,
B représente hydrogène, et
G⁴ représente le radical (E) indiqué dans la revendication 1.

11. Agent, **caractérisé par** une teneur en au moins un composé de formule (I) selon les revendications 1 à 9.

12. Procédé de lutte contre des organismes nuisibles, **caractérisé en ce qu'**un composé de formule (I) selon les revendications 1 à 10 ou un agent selon la revendication 11 est laissé agir sur les organismes nuisibles et/ou leur habitat, les procédés sur le corps animal ou humain étant exclus.

13. Composés de formule (XII) 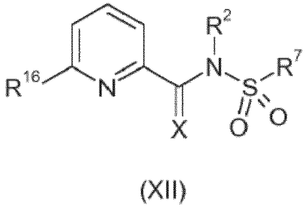 dans laquelle
R¹⁶ représente fluor, chlore, brome ou iode,
X et R² ont les significations indiquées dans la revendication 1, et
R⁷ représente un radical de la série constituée par alkyle en C₁-C₆, alcényle en C₂-C₆ et alcynyle en C₂-C₆, chacun éventuellement substitué par halogène, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆ et halogénoalkylsulfonyle en C₁-C₆; cycloalkyle en C₃-C₆, cycloalkyle en C₃-C₆-alkyle en C₁-C₆ et cycloalcényle en C₃-C₆, chacun éventuellement substitué par halogène, alkyle en C₁-C₆, halogénoalkyle en C₁-C₆, alcoxy en C₁-C₆ ou halogénoalcoxy en C₁-C₆, dans lesquels les cycles peuvent contenir au moins un hétéroatome de la série constituée par soufre, oxygène (des atomes d'oxygène ne pouvant pas être directement voisins) et azote ; hétéroaryle et hétéroaryl-alkyle en C₁-C₆, chacun éventuellement substitué par halogène, cyano (également dans la partie alkyle), nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, amino, alkylamino en C₁-C₆, di(alkyle en C₁-C₆)amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle ; ou NR'R",
R' et R" représentant chacun indépendamment l'un de l'autre un radical de la série constituée par hydrogène, alkyle en C₁-C₆, haloalkyle en C₁-C₆, cycloalkyle en C₃-C₆, cycloalkyle en C₃-C₆-alkyle en C₁-C₆, alcoxy en C₁-C₆, alkylcarbonyle en C₁-C₆ et alcoxycarbonyle en C₁-C₆, et hétéroaryle ayant les significations indiquées dans la revendication 1.

14. Composés de formule (XV) 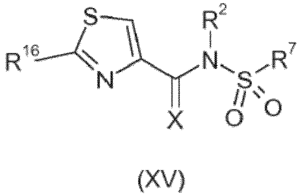 dans laquelle
R¹⁶ représente fluor, chlore, brome ou iode, et
X et R² et R⁷ ont les significations indiquées dans la revendication 1, et
R⁷ représente un radical de la série constituée par alkyle en C₁-C₆, alcényle en C₂-C₆ et alcynyle en C₂-C₆, chacun éventuellement substitué par halogène, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆ et halogénoalkylsulfonyle en C₁-C₆; cycloalkyle en C₃-C₆, cycloalkyle en C₃-C₆-alkyle en C₁-C₆ et cycloalcényle en C₃-C₆, chacun éventuellement substitué par halogène, alkyle en C₁-C₆, halogénoalkyle en C₁-C₆, alcoxy en C₁-C₆ ou halogénoalcoxy en C₁-C₆, dans lesquels les cycles peuvent contenir au moins un hétéroatome de la série constituée par soufre, oxygène (des atomes d'oxygène ne pouvant pas être directement voisins) et azote ; hétéroaryle, aryl-alkyle en C₁-C₆ et hétéroaryl-alkyle en C₁-C₆, chacun éventuellement substitué par halogène, cyano (également dans la partie alkyle), nitro, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, amino, alkylamino en C₁-C₆, di(alkyle en C₁-C₆)amino, alkylcarbonylamino en C₁-C₆, alcoxycarbonylamino en C₁-C₆, alcoxy en C₁-C₆-alkyle en C₁-C₆, halogénoalcoxy en C₁-C₆-alkyle en C₁-C₆, alcényle en C₂-C₆, alcynyle en C₂-C₆, cycloalkyle en C₃-C₆-alkyle en C₁-C₆, alkylcarbonyle en C₁-C₆, alcoxycarbonyle en C₁-C₆ ou aminocarbonyle ; ou NR'R",
R' et R" représentant chacun indépendamment l'un de l'autre un radical de la série constituée par hydrogène, alkyle en C₁-C₆, haloalkyle en C₁-C₆, cycloalkyle en C₃-C₆, cycloalkyle en C₃-C₆-alkyle en C₁-C₆, alcoxy en C₁-C₆, alkylcarbonyle en C₁-C₆ et alcoxycarbonyle en C₁-C₆, et aryle et hétéroaryle ayant les significations indiquées dans la revendication 1.

15. Composés de formule (XVI) 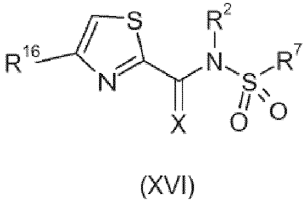 dans laquelle
R¹⁶ représente fluor, chlore, brome ou iode, et
X et R² et R⁷ ont les significations indiquées dans la revendication 1.
